# EUROPEAN PATENT SPECIFICATION

(11) **EP 2 412 814 B1**
(45) Date of publication and mention of the grant of the patent: **10.08.2016**
(21) Application number: 10714044.4
(22) Date of filing: 17.03.2010
(51) Int. Cl.: C12N 15/82, A01H 5/00

(54) **Method for the production of transgenic plants having high starch and biomass content and yield**
Verfahren zur Herstellung transgener Pflanzen mit hohem Stärke- und Biomassengehalt und -ertrag
Procédé pour la production de plantes transgéniques qui présentent une teneur et un rendement élevés en amidon et biomasse

(30) Priority: 24.03.2009 ES 200930009
(43) Date of publication of application: 01.02.2012
(73) Proprietor: Iden Biotechnology, 31002 Pamplona (ES)
(72) Inventor: MUÑOZ PÉREZ, Francisco José, E-31002 Pamplona (ES); LI, Jun, E-31002 Pamplona (ES); POZUETA-ROMERO, Javier, E-31002 Pamplona (ES); RAYNAUD, Sandy, E-31002 Pamplona (ES); RAGEL DE LA TORRE, Paula, E-31002 Pamplona (ES); MÉRIDA BERLANGA, Ángel, E-31002 Pamplona (ES); BAROJA, Miren Edurne, E-31002 Pamplona (ES); MONTERO, Manuel, E-31002 Pamplona (ES)
(74) Representative: Illescas, Manuel
(86) International application number: PCT/ES2010/070158
(87) International publication number: WO 2010/109045

(56) References cited:
- WO-A1-2008/012356
- US-B2- 6 734 341
- US-B2- 6 849 781
- ROLDAN I ET AL: "The phenotype of soluble starch synthase IV defective mutants of Arabidopsis thaliana suggests a novel function of elongation enzymes in the control of starch granule formation" PLANT JOURNAL FEBRUARY 2007 BLACKWELL PUBLISHING LTD GB LNKD- DOI:10.1111/J.1365-313X.2006.02968.X, vol. 49, no. 3, February 2007 (2007-02), pages 492-504, XP002585103 cited in the application
- DIAN WEIMIN ET AL: "Evolution and expression analysis of starch synthase III and IV in rice" JOURNAL OF EXPERIMENTAL BOTANY, OXFORD UNIVERSITY PRESS, GB LNKD- DOI:10.1093/JXB/ERI065, vol. 56, no. 412, 1 February 2005 (2005-02-01), pages 623-632, XP002403602 ISSN: 0022-0957
- SZYDLOWSKI NICOLAS ET AL: "Starch Granule Initiation in Arabidopsis Requires the Presence of Either Class IV or Class III Starch Synthases" PLANT CELL, vol. 21, no. 8, August 2009 (2009-08), pages 2443-2457, XP002585102 ISSN: 1040-4651
- YODER DAVID W ET AL: "Effects of mutations in arabidopsis FtsZ1 on plastid division, FtsZ ring formation and positioning, and FtsZ filament morphology in vivo", PLANT AND CELL PHYSIOLOGY, vol. 48, no. 6, June 2007 (2007-06), pages 775-791, ISSN: 0032-0781
- STOKES KEVIN D ET AL: "Chloroplast division and morphology are differentially affected by overexpression of FtsZ1 and FtsZ2 genes in Arabidopsis", PLANT PHYSIOLOGY (ROCKVILLE), vol. 124, no. 4, December 2000 (2000-12), pages 1668-1677, ISSN: 0032-0889

## Description

### FIELD OF THE INVENTION

The present invention is in the fields of genetic engineering and plant physiology. Specifically, the invention comprises a process for obtaining transgenic plants with a high content and yield of starch and biomass; it also includes transformed plant cells, the transgenic plants obtained by this process and their uses.

### STATE OF THE ART

The main ways of storing reserve carbohydrates are glycogen (in animals and bacteria) and starch (in plants). In plants, the starch accumulates in large quantities in organs such as seeds and tubers and is a fundamental constituent of the human diet. Starch is also a renewable and fully biodegradable material, often used in the paper, cosmetics, pharmaceutical and food industries, as well as being used as a fundamental component for the manufacture of biodegradable plastics, paints with low environmental impact and bioethanol.

The biosynthesis of starch is a complex process requiring the concerted action of various enzyme activities such as sucrose synthase, phosphoglucomutase, ADP-glucose pyrophosphorylase and various types of glucosyltransferases, commonly named starch synthases (SS) and starch ramification and deramification enzymes (1).

SS in plants and glycogen synthase (GlgA) in bacteria catalyse the transfer of the glucosidic half of the ADP-Glucose molecule (the activated donor of glucose) to a pre-existing α(1, 4)-glucan. The same SSs have been found in all photosynthetic organisms that accumulate starch and are denominated: SSI, SSII, SSIII, SSIV and GBSSI. This high degree of conservation indicates that each of these proteins performs different functions in the process of creation of a starch granule (2). Thus, for example, GBSSI is involved in the production of amylose, while SSI, SSII and SSIII are involved in the production of short starch chains and medium and long amylopectin chains respectively (3).

SSIV is the least known of the protein family known as soluble SSs. Its amino acid sequence is between 30% and 50% different from that of SSI, SSII and SSIII (4, 5). Despite its name, its SS activity has still not been demonstrated. Furthermore, the idea has recently arisen that SSIV does not cover the field of action and the function of the other SSs (6). However, there is evidence to suggest that SSIV can be involved in the determination of the number of starch granules in the plastid (7).

There are numerous references showing that the reduction in the activity of SSI, SSII and SSIII brings about a reduction in starch levels and a change in the structure and composition of the granule (8, 9). Mutants of Arabidopsis without SSIV accumulate reduced levels of starch because, although the amylose/amylopectin balance and molecular structure of the amylopectin are normal, they only produce one granule of starch per chloroplast (7).

In contrast to what was hoped, transgenic plants over-expressing GlgA of Escherichia coli accumulate low starch content (10). Although ectopic expression of SSI, SSII and SSIII has been used as a strategy to increase starch content (WO 00/66745) and modify the properties of starch such as the phosphate content (WO2007/009823) (11-13) and the amylose/amylopectin balance (WO 2006/084336; WO 2002/018606), there is no experimental evidence to indicate that SSIV has SS activity or that the ectopic expression of SSIV can be used as a biotechnological strategy to increase starch content, yield and biomass accumulation in plants. In the present invention, after demonstrating that SSIV is a SS with properties that are totally different to those of the soluble SSs (SSI, SSII, SSIII), we describe for the first time that over-expression of SSIV is a biotechnological strategy for the production of transgenic plants with high levels of starch and high yields of starch and biomass.

### DESCRIPTION OF THE INVENTION

### Brief description of the invention

The present invention refers to a process for obtaining transgenic plants with a content of starch at least 10% higher than the content found in non-transformed plants (WT), characterized by the transformation of non-transformed plants with an expression vector comprising a nucleotide sequence encoding and over-expressing for an enzyme with SSIV activity represented by SEQ ID NO:4. In a preferred embodiment, the transgenic plants obtained contain at least 10% biomass higher than non-transformed plants (WT).The present invention also refers to the transgenic plants characterised by these properties.

The technical effects shown in the present invention can be extrapolated to any type of plant organ such as tubers, leaves, fruit and seeds as well as to any type of plant such as, for example: Arabidopsis, potato, tobacco, tomato, rice, barley, wheat and corn. The results shown in the present invention were achieved for AtSSIV, the gene coding for SSIV in A. thaliana, expressed both constitutively under the control of the 35S promoter and also under the control of a specific tuber promoter (the potato gene promoter). It should be noted that constitutive expression was particularly preferred. The results shown in the present invention were achieved after over-expressing any SSIV isoform and sequence (the particularly preferred from was that of Arabidopsis SSIV). That is, any promoter that is expressed in plants and produces over-expression of either AtSSIV or any other isoform of SSIV are encompassed by the present invention.

For the purpose of the present invention, the following terms are defined:
- Transgenic plant: plant where the genome has been modified by genetic engineering with the aim of obtaining different and/or improved biological properties compared to the wild control plant (WT) when both are cultivated under the same conditions.
- Transformed plant cell: are plant cells with a genetic alteration resulting from the introduction and expression of genetic material that is external to its genome.
- Over-expression of SSIV: a plant over-expresses the SSIV enzyme when the intensity of the band obtained in a Western Blot of a transformed plant extract is significantly higher than that of an extract of a WT plant cultivated in the same conditions and at the same time.
- High starch content: as used in the present invention, this expression directly refers to a statistically significant value that is at least 10% higher than values found in control plants.
- High biomass productivity: as used in the present invention, this expression directly refers to a statistically significant increase, which is defined as the increase in fresh weight of transgenic plants during their development that is faster than that of wild plants.
- SSIV activity: The activity of the SSIV enzyme consists in transferring units of glucose from ADP-Glucose to maltotriose and polyglucans such as starch, amylose, amylopectin and glycogen.

### Brief description of the figures

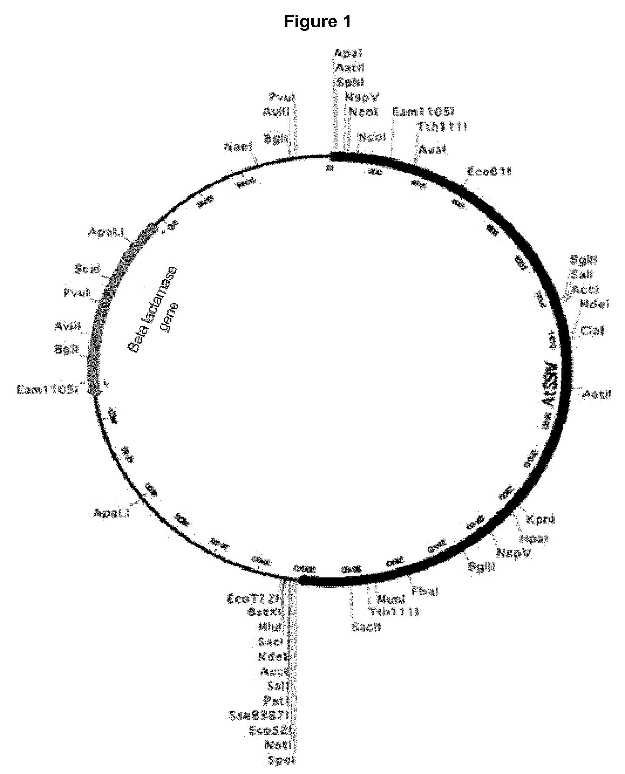

**Figure 1****.** Restriction map of the pAtSSIV plasmid resulting from the cloning of a complete cDNA coding for the Arabidopsis thaliana AtSSIV gene in the pGEM-T easy (Promega) vector.

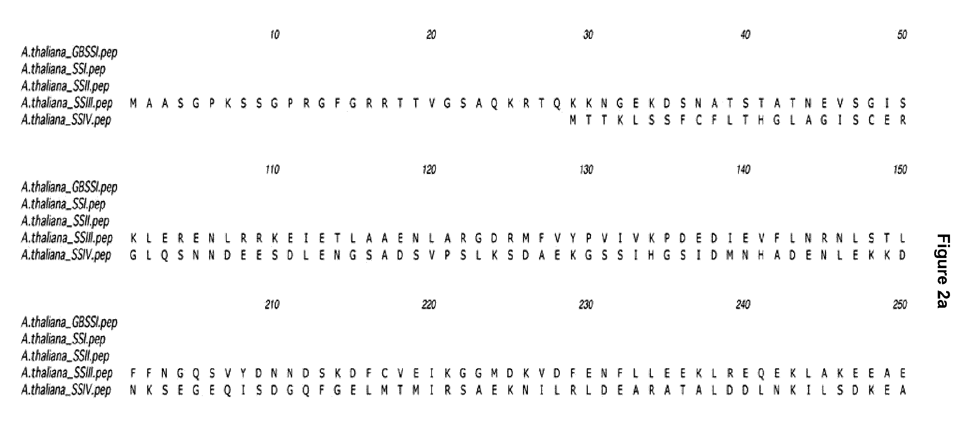

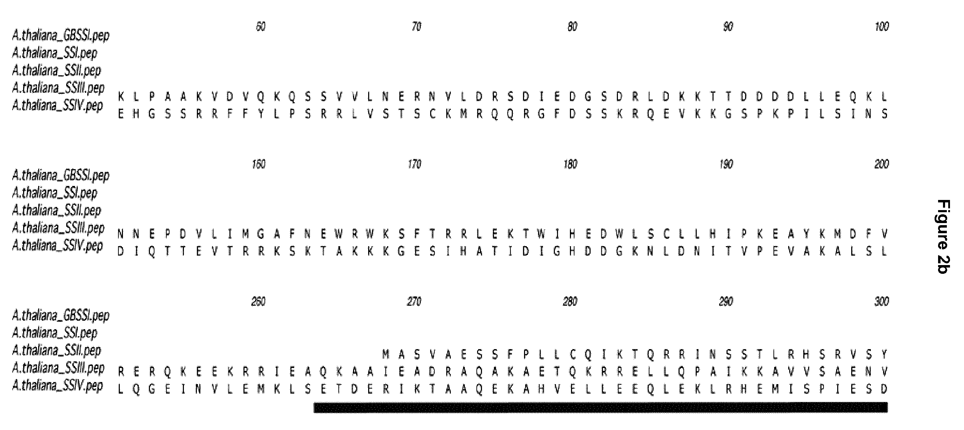

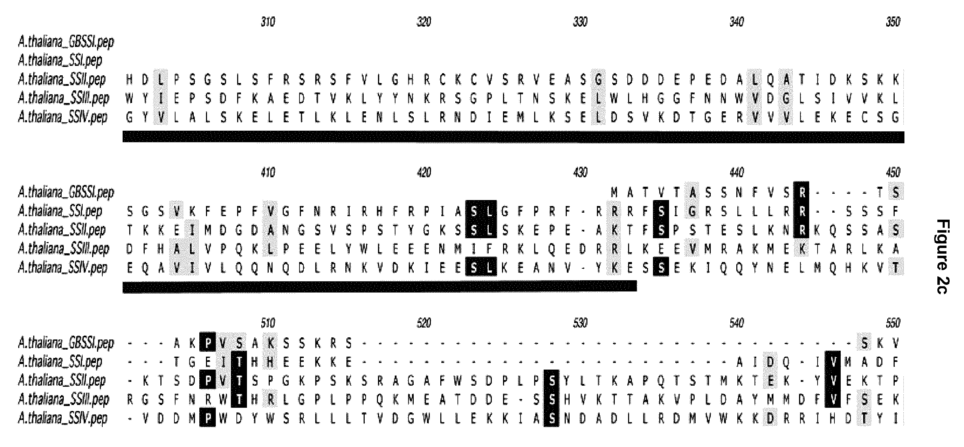

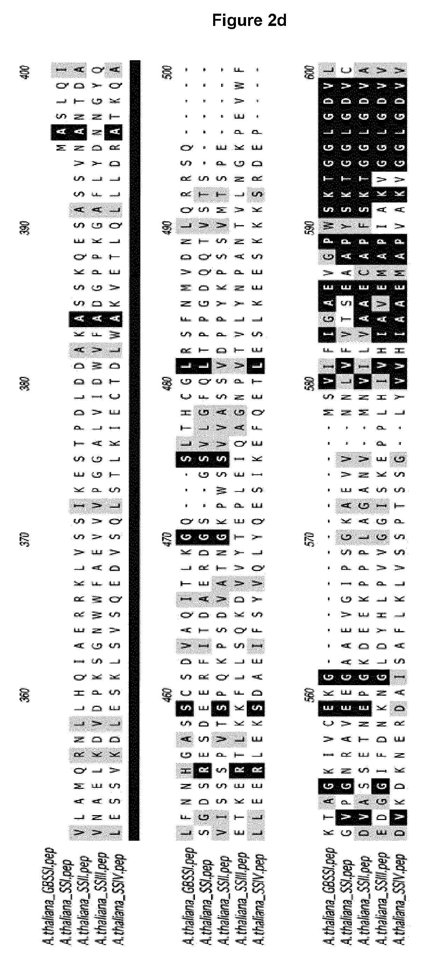

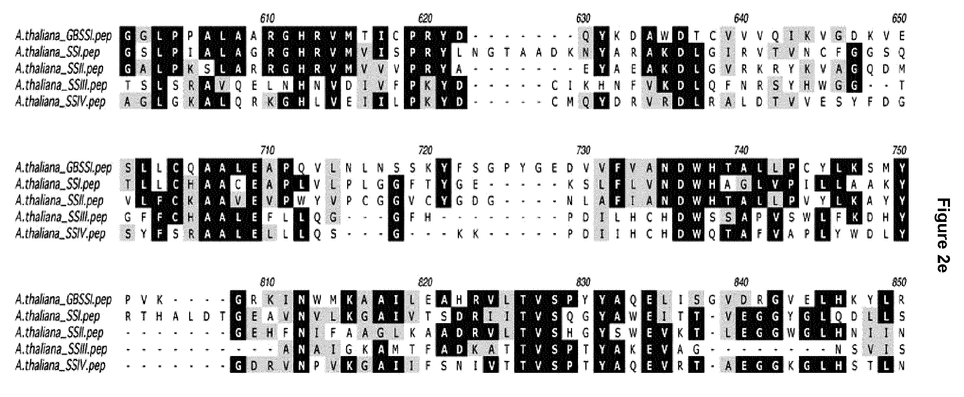

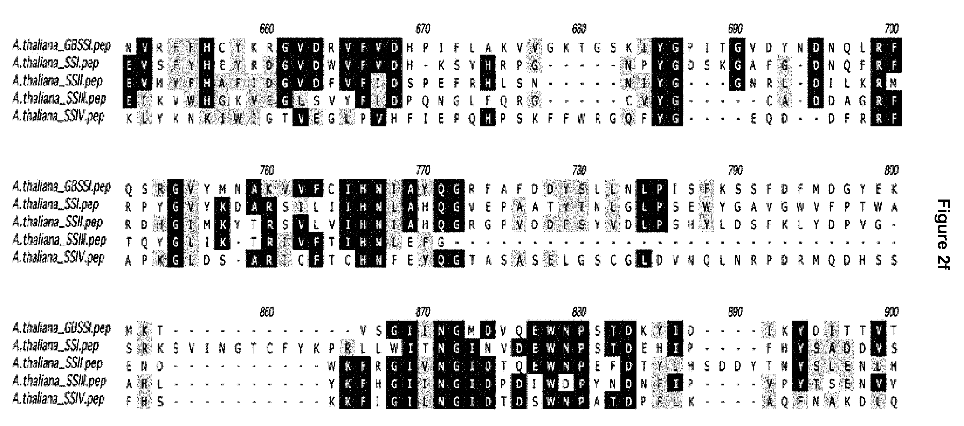

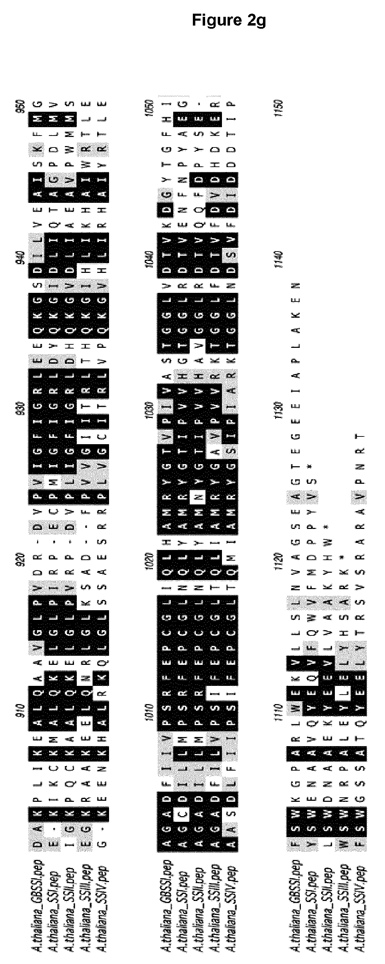

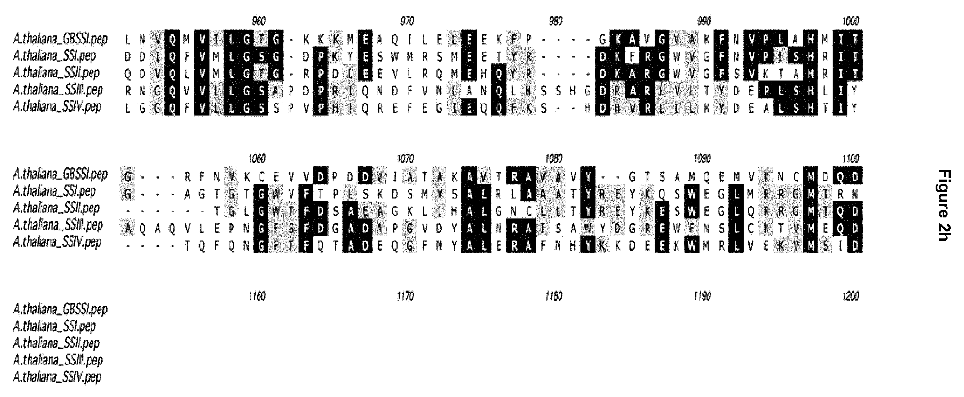

**Figure 2****.** (a-h) Comparison of the amino acid sequences of AtSSI, AtSSII, AtSSIII and AtSSIV. The amino acids that are conserved in all the SSs are highlighted in black. The fragment of AtSSIV used to obtain specific antibodies against this protein is indicated with a bold black line.

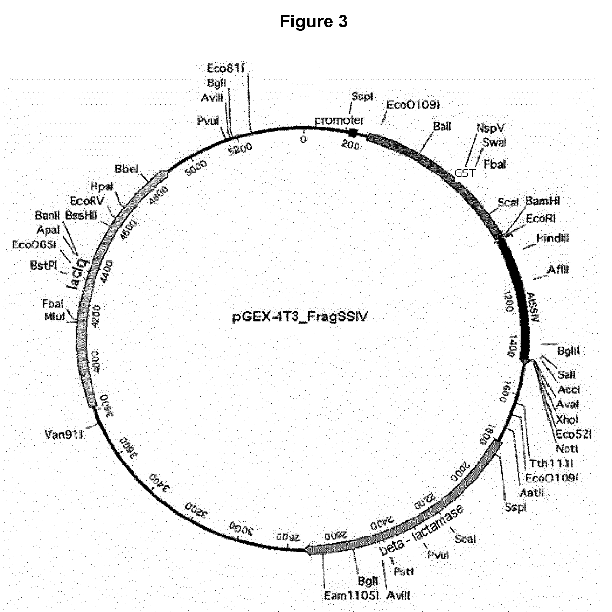

**Figure 3****.** Restriction map of the pGEX-4T3_FragSSIV plasmid used for the synthesis of the peptide necessary for the production of the specific antibody against SSIV.

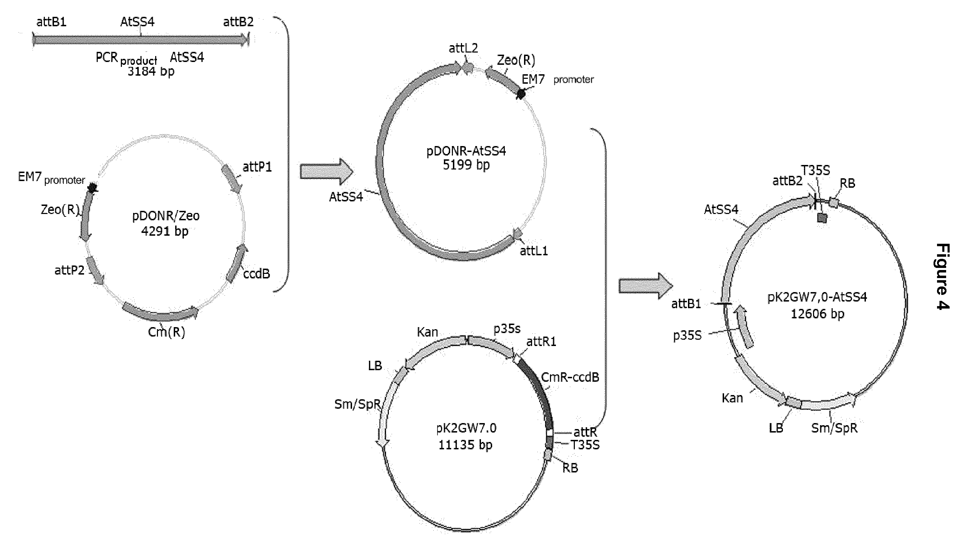

**Figure 4****.** Stages of the construction of the pK2GW7,0-AtSSIV binary plasmid (alternatively designated pKan-35S-AtSSIV) necessary for the transformation of plants with Agrobacterium tumefaciens.

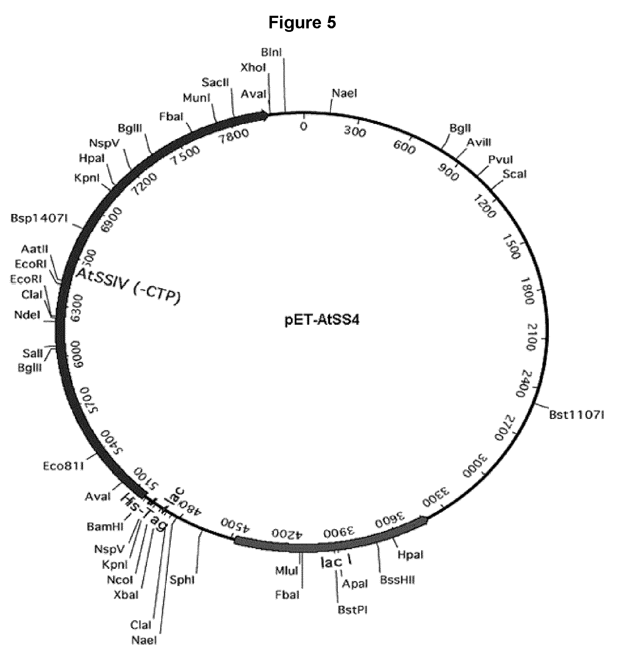

**Figure 5****.** Restriction map of the pET-AtSSIV plasmid necessary for expression of mature SSIV in E. coli.

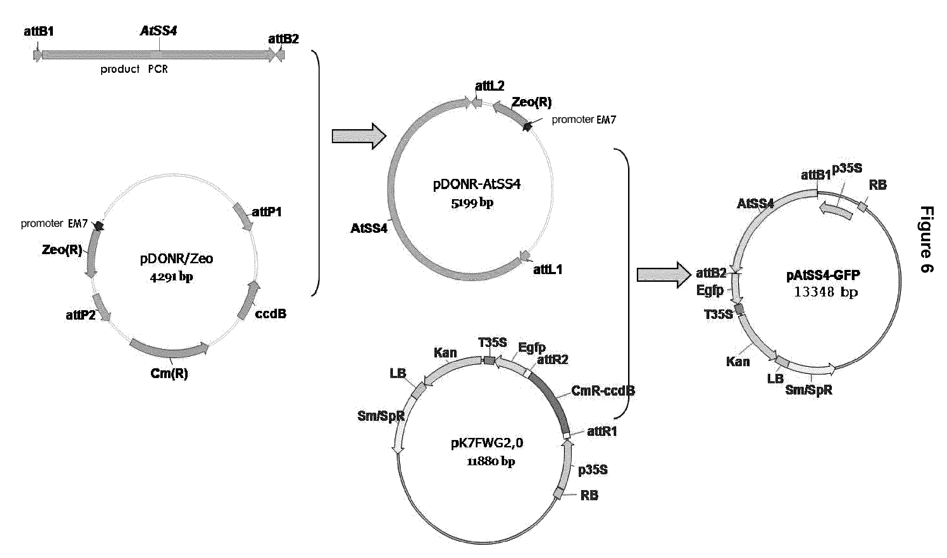

**Figure 6****.** Stages in the construction of the pAtSSIV-GFP binary plasmid necessary for the transformation of plants with Agrobacterium tumefaciens.

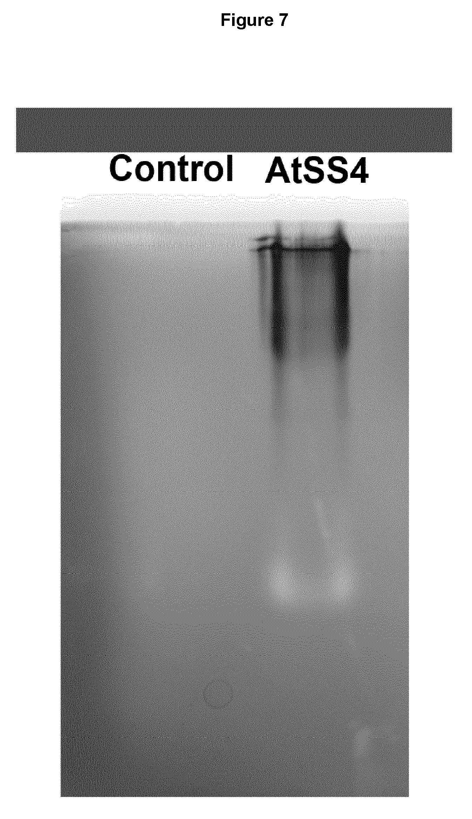

**Figure 7****.** Zymogram of SS activity using glycogen as substrate. The SSIV enzyme is separated by electrophoresis in a gel containing glycogen. To provide the signal shown in the figure, the gel was incubated in a solution with ADP-Glucose and later in Lugol's solution, giving rise to the dark band shown. The staining is due to the affinity of Lugol's solution for long chain glucose polymers.

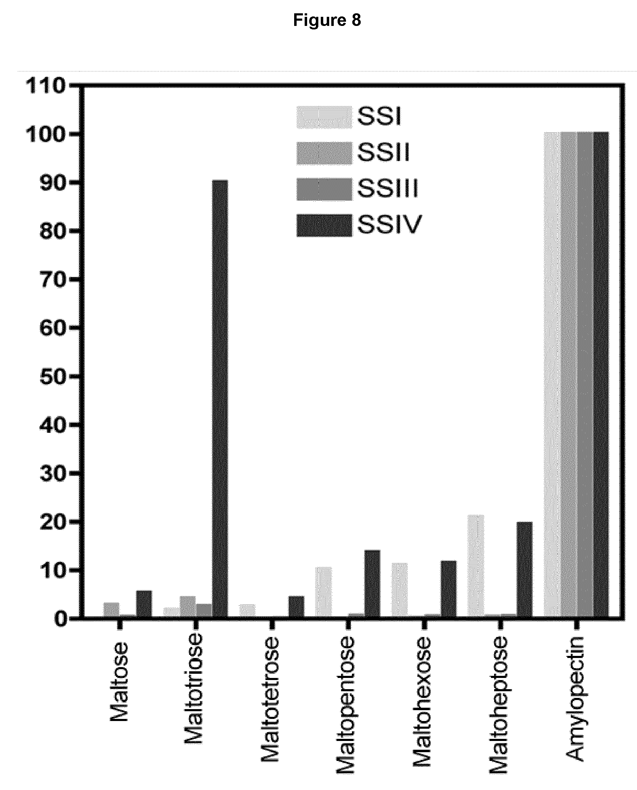

**Figure 8****.** Specificity of the substrate. In vitro assay of SSI, SSII, SSIII and SSIV with different malto-oligosaccharides as substrates.

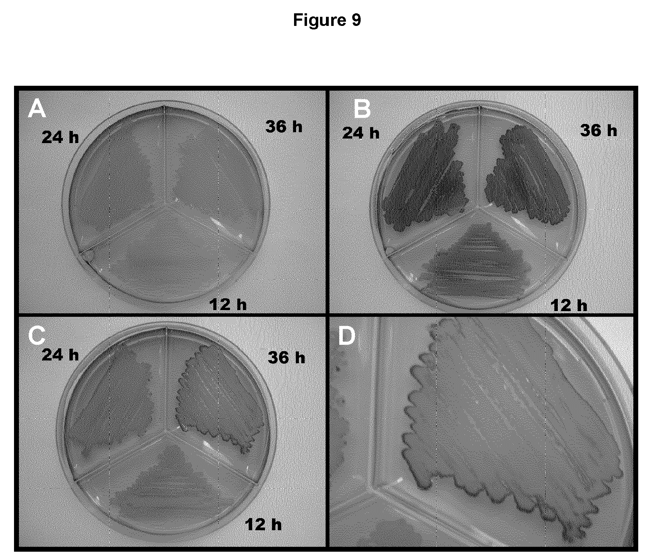

**Figure 9****.** SSIV is capable of complementing the "glycogen-less" phenotype of AgIgAP cells. Template of iodine staining after 12, 24 and 36 hours of incubation of (A) AgIgAP, (B) AgIgAP expressing GIgA and (C, D) AgIgAP expressing SSIV. Figure D shows an amplification of the iodine stain pattern after 36 hours incubation of AgIgAP cells expressing SSIV.

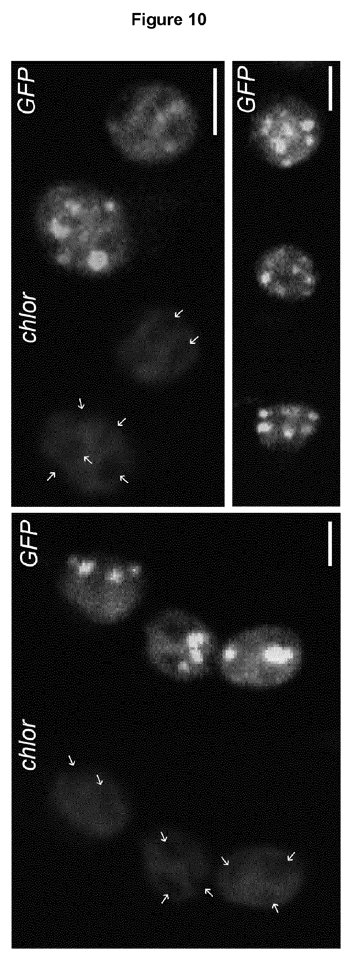

**Figure 10****.** Subcellular localisation of AtSSIV. The illustration shows the fluorescence produced in Arabidopsis plant cells transformed with AtSSIV-GFP that have been subjected to analysis by a D-Eclipse C1 (NIKON) confocal microscope equipped with an Ar 488 excitation laser, a BA515/30 filter for green emission, a BA650LP filter for red emission and a light detector. In the photographs it can been seen (arrows) that SSIV-GFP is located on the surface of starch grains. chlor: chlorophyll; GFP: "Green Fluorescent Protein", is the fluorescent protein fused to SSIV capable of emitting fluorescence that is viewed in the confocal microscope.

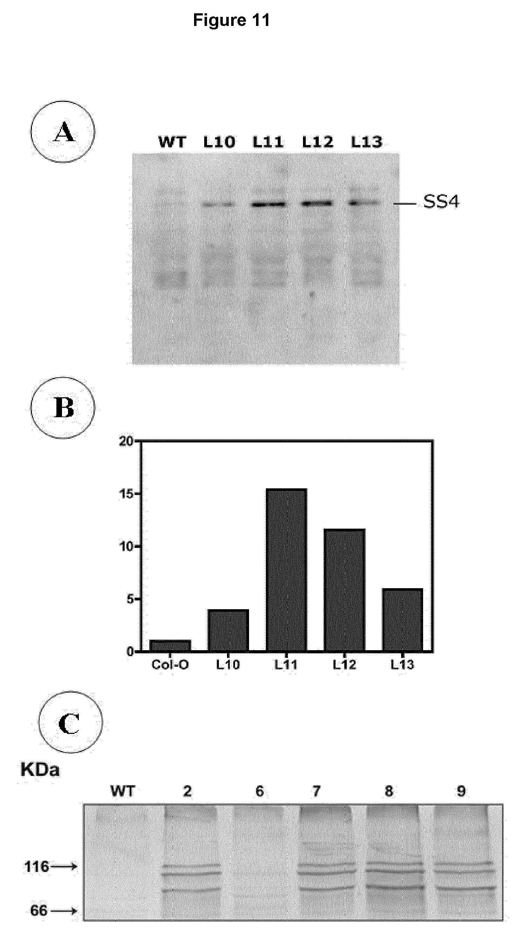

**Figure 11****.** Analysis by Western Blot (A) and quantification (B) of the levels of SSIV protein in the wild ecotype Col-0 (WT) and in the transgenic lines that over-expressed the AtSSIV gene (L10, L11, L12 and L13) after integrating the 35S-AtSSIV construction into their genome making use of the A. tumefaciens DSM 19675 strain. In (C), analysis by Western Blot of SSIV in potato tubers expressing AtSSIV after integrating the 35S-AtSSIV construction into their genome making use of the A. tumefaciens DSM 19675 strain. Transgenic plants are labelled 2, 6, 7, 8 and 9.

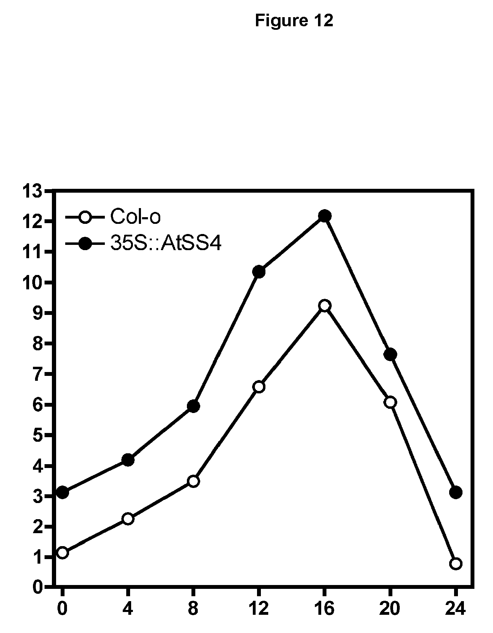

**Figure 12****.** Levels of starch in Arabidopsis plant leaves cultivated in greenhouse conditions with a cycle of 16 hours light/8 hours darkness. The white circles correspond to wild Arabidopsis thaliana plants, ecotype Col-0. The black circles correspond to transgenic Arabidopsis plants over-expressing the AtSSIV gene coding for Arabidopsis thaliana SSIV.

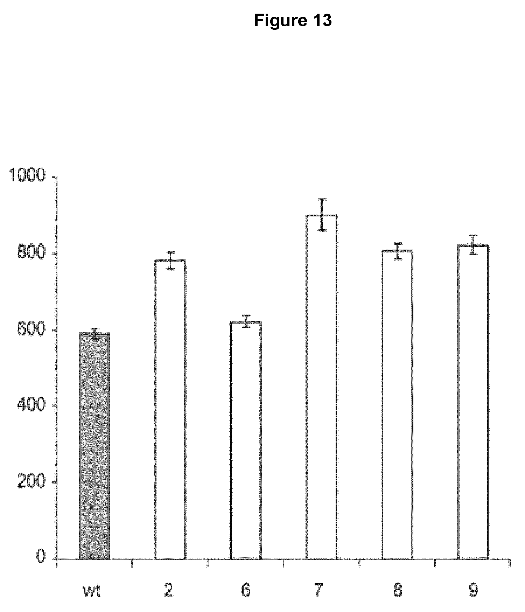

**Figure 13****.** Starch content in tubers of wild potato plants and potato plants expressing AtSSIV after integrating the 35S-AtSSIV construction into their genome making use of the A. tumefaciens DSM 19675 strain, cultivated in greenhouse conditions. The wild tubers analysed are labelled as WT. The transgenic plants are labelled 2, 6, 7, 8 and 9. The values shown correspond to the average and standard deviation of 10 different plants per line.

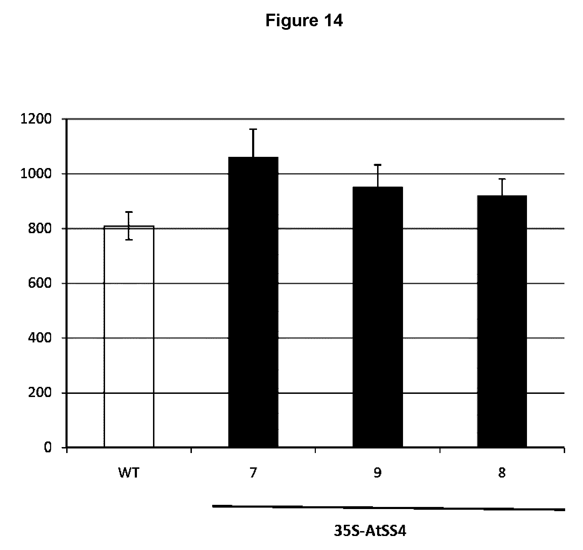

**Figure 14****.** Starch content in tubers of wild potato plants and potato plants expressing AtSSIV after integrating the 35S-AtSSIV construction into their genome making use of the A. tumefaciens DSM 19675 strain, cultivated in field conditions. The wild tubers analysed are labelled as WT. The transgenic plants are labelled 7, 8 and 9. The values shown correspond to the average and standard deviation of 30 different plants per line.

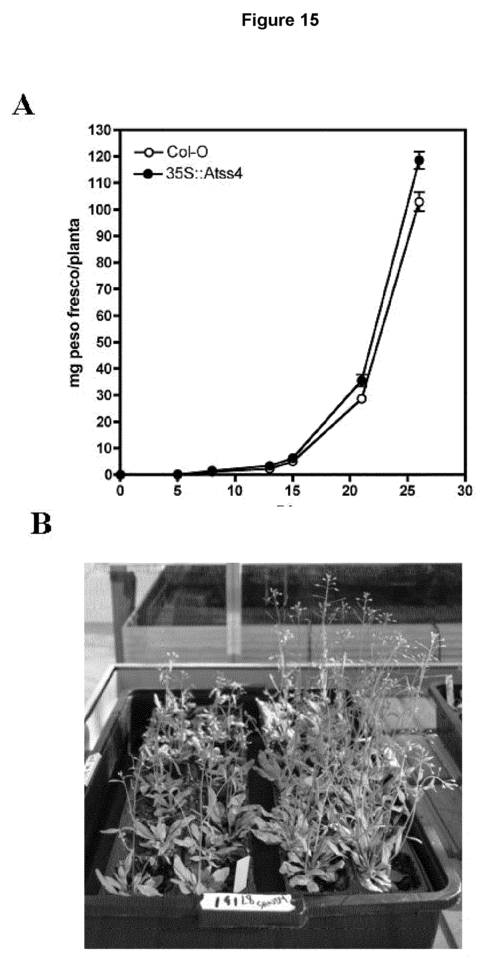

**Figure 15**. (A) Change in fresh weight of wild Arabidopsis thaliana plants, ecotype Col-0 (white circles) and transgenic Arabidopsis plants over-expressing the AtSSIV gene (black circles), both cultivated in greenhouse conditions throughout their growth phase. The data are the average of three measurements. Each measurement was made by weighing the above-ground parts of 5 plants and dividing the value obtained by five. The bars indicate the standard deviation of the measurements. (B) Visual comparison of Arabidopsis ecotype Col-0 plants (left side of the photograph) and transgenic Arabidopsis plants over-expressing the AtSSIV gene (right side of the photograph).

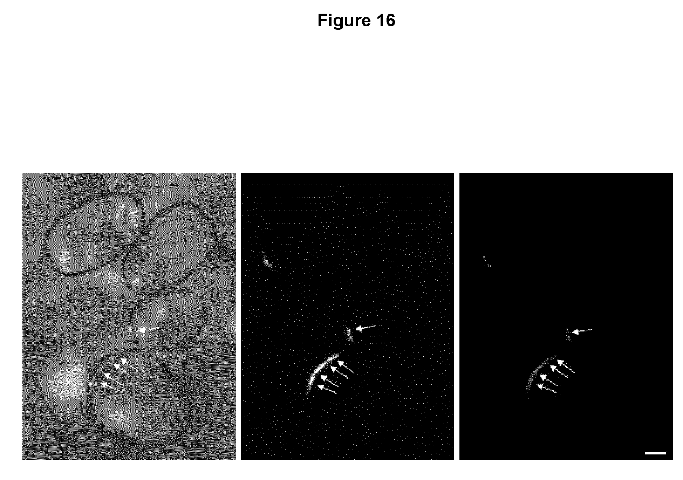

**Figure 16****.** Subcellular localisation of AtSSIV in amyloplasts of potato plant tubers over-expressing AtSSIV after integrating the p35S-AtSSIV construction into their genome making use of the A. tumefaciens DSM 19675 strain, cultivated in field conditions. The illustration shows the fluorescence produced in potato plant tubers transformed with AtSSIV-GFP that have been analysed by a D-Eclipse C1 (NIKON) confocal microscope equipped with an Ar 488 excitation laser, a BA515/30 filter for green emission, a BA650LP filter for red emission and a light detector. In the photographs, it can be seen that SSIV-GFP is located at the poles of the starch granules of the tubers of these transgenic plants (indicated by arrows). White bar: 5 µm.

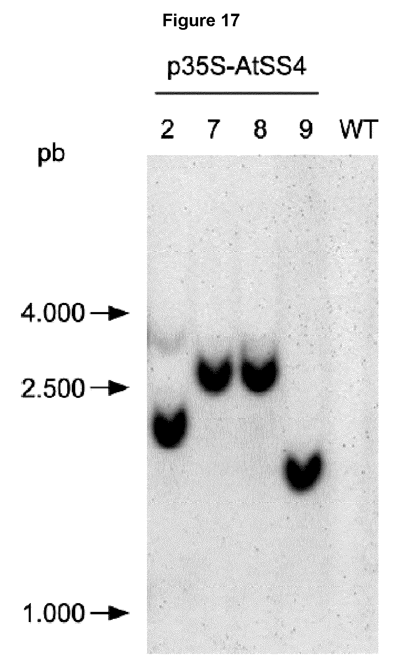

**Figure 17****.** Southern Blot of transgenic potato plants over-expressing AtSSIV after integrating the p35S-AtSSIV construction into their genome making use of the A. tumefaciens DSM 19675 strain. The figure shows the presence of a single insertion of the p35S-AtSSIV construction in these plants. Non-transformed plants (WT) do not show this construction in their genome. The transgenic plants belong to different lines: 2, 7, 8 and 9.

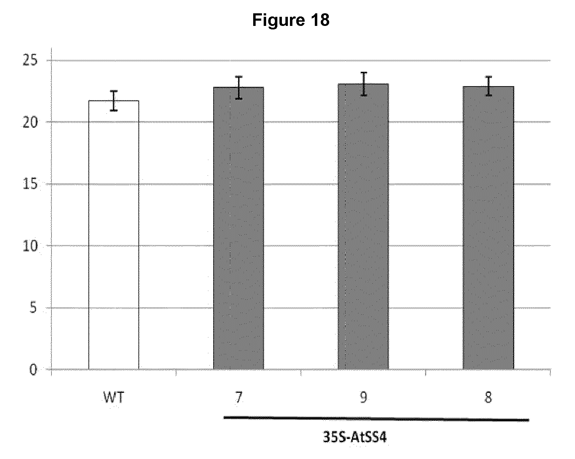

**Figure 18****.** Amylose/Amylopectin balance expressed as % amylose in non-transformed control potato plant tubers (WT) and potato plants over-expressing AtSSIV after integrating the p35S-AtSSIV construction into their genome making use of the A. tumefaciens DSM 19675 strain. The data shown in the figure are for plants cultivated in field conditions. The values shown are the average and the standard deviation of the tubers of 30 different plants per line.

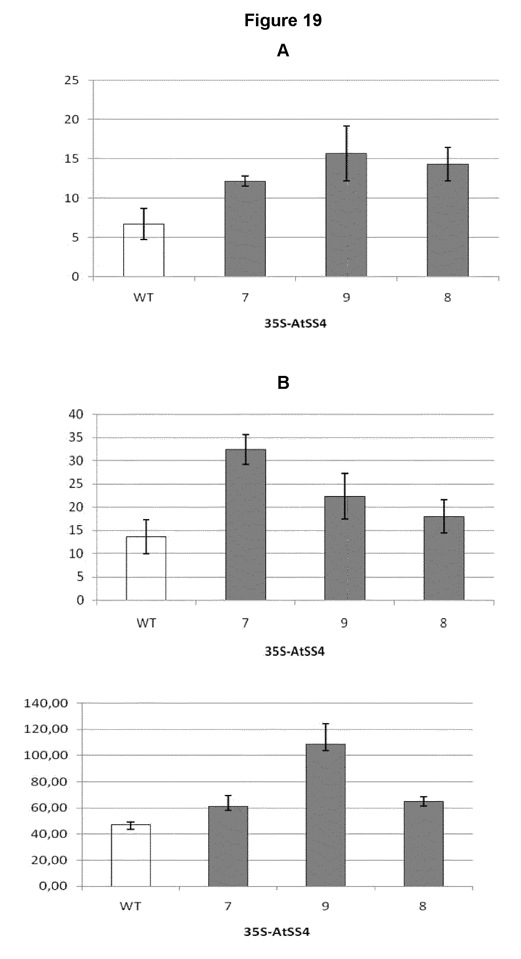

**Figure 19****.** Glucose (A), fructose (B) and sucrose (C) content in non-transformed control potato plant tubers (WT) and in potato plants over-expressing AtSSIV after integrating the p35S-AtSSIV construction into their genome making use of the A. tumefaciens DSM 19675 strain. The values shown correspond to the average and standard deviation of 30 different plants per line, cultivated in field conditions. The concentration of each of the sugars is expressed as µmol/g tuber fresh weigh.

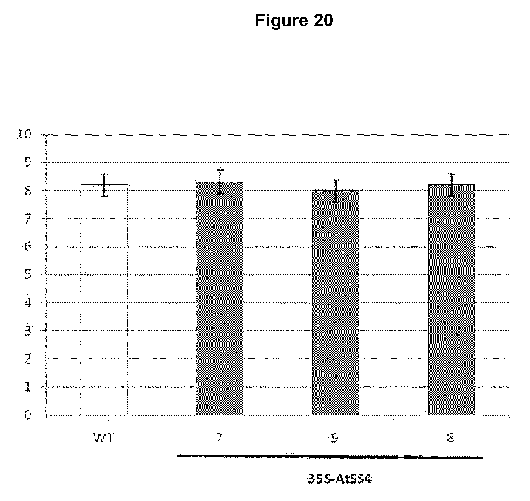

**Figure 20****.** Protein content in non-transformed control potato plant tubers (WT) and in potato plants over-expressing AtSSIV after integrating the p35S-AtSSIV construction into their genome making use of the A. tumefaciens DSM 19675 strain. The values shown correspond to the average and standard deviation of 30 different plants per line, cultivated in field conditions. The protein concentration is expressed as mg/g tuber fresh weight.

### Detailed description of the invention

One of the objects described in the present invention refers to the process for obtaining transgenic plants with a content of starch at least 10% higher than the content found in non-transformed plants (WT), characterised by the transformation of non-transformed plants (WT) with an expression vector comprising a nucleotide sequence encoding and over-expressing for an enzyme with SSIV activity represented by SEQ ID NO:4. In a preferred embodiment, the transgenic plants obtained contain at least 10% biomass higher than non-transformed plants (WT).

In a preferred embodiment, the process of the invention is characterised in that the level of SSIV expression inside the transformed plant is at least twice the level of SSIV expression in the non-transformed plant.

In another preferred embodiment, the process of the invention is characterised in that the enzyme with SSIV activity represented by SEQ ID NO:4 is encoded by a nucleotide sequence comprised in the expression vector used for transforming the non-transformed plant (WT), which comprises SEQ ID NO: 3.

Another object of the present invention refers to the cells transformed with an expression vector, preferably a plasmid, that comprises a nucleotide sequence encoding and over-expressing for a protein with SSIV activity represented by SEQ ID NO:4 and having a content of starch at least 10% higher than the content found in non-transformed plant (WT) cells. In a preferred embodiment, the plant cells contain at least 10% biomass higher than non-transformed plants (WT).

In a preferred embodiment, the cells of the invention are characterised in that the non-transformed plant (WT) cells are transformed with an expression vector comprising a nucleotide sequence which comprises SEQ ID NO: 3 selected from: Agrobacterium tumefaciens DSM 19675, plasmid pET-AtSSIV or plasmid pGEX-4T3_FragSSIV, preferably with the Agrobacterium tumefaciens DSM 19675 expression vector. These cells also belong to any of the following plant species: potato (Solanum tuberosum), tobacco (Nicotiana tabacum), barley (Hordeum vulgare), rice (Oryza sativa), corn (Zea mays) or Arabidopsis (Arabidopsis thaliana).

Another object of the present invention refers to transgenic plants characterised in that they are transformed with an expression vector that comprises a nucleotide sequence encoding and over-expressing for a protein with SSIV activity represented by SEQ ID NO:4, and having a content of starch at least 10% higher than the content found in non-transformed plants (WT). In a preferred embodiment, the transgenic plant contains at least 10% biomass higher than non-transformed plants (WT).

In a preferred embodiment, the transgenic plants of the invention are characterised by showing a level of expression of SSIV that is at least twice that observed in the non-transformed plants (WT).

In another preferred embodiment, the transgenic plants of the invention are characterized in that the nucleotide sequence, comprised in the expression vector used for transforming the non-transformed plant (WT), comprises SEQ ID NO:3.

In another preferred embodiment, the transgenic plants of the invention are characterised in that they are selected from a group comprising: potato (Solanum tuberosum), tobacco (Nicotiana tabacum), barley (Hordeum vulgare), rice (Oryza sativa), corn (Zea mays) or Arabidopsis (Arabidopsis thaliana).

Another object of the present invention refers to the use of the transgenic plants; or the transformed plant cells described above, for the production of carbohydrates, selected from a group including starch, glucose, fructose and sucrose, and also for the production of biomass.

### Obtaining cDNA coding for SSIV

AtSSIV is coded for by the At4g18240 (or AtSSIV) gene. Starting from its nucleotide sequence, specific oligonucleotides were synthesised for the AtSSIV gene. These oligonucleotides were used for RT-PCR amplification of the complete fragment of cDNA coding for AtSSIV, starting from the total RNA of Arabidopsis leaves. The amplified fragment was cloned into the pGEM-T easy (Promega) vector giving rise to the pAtSSIV plasmid (Figure 1) that was amplified in the XL1-Blue host bacterium.

### Obtaining specific polyclonal antibodies against the AtSSIV protein

A fragment of the amino terminal region of the protein not showing homology with the other SSs in Arabidopsis (Figure 2) was selected as the antigenic fragment for obtaining a polyclonal antibody against AtSSIV. Specifically, the region between amino acids Glutamic 236 and Glutamic 414 of the AtSSIV amino acid sequence was used. The oligonucleotides characterised by SEQ ID NO: 5 and 6 were used for cloning the cDNA sequence coding for this fragment.

The 512 base-pair fragment was amplified by PCR using these oligonucleotides and cDNA (SEQ ID NO: 3) of the first chain obtained from mRNA of leaves as primers. The oligonucleotides introduce restriction sites for the Ndel and Xhol enzymes at the 5' and 3' ends respectively of the amplified fragment. These were used for cloning the cDNA fragment into the pGEX-4T (Amersham Biosciences) expression vector, giving rise to the pGEX-4T3_FragSSIV plasmid (Figure 3). This expression vector contains the sequence coding for the glutathione S-transferase (GST) protein. The cloning of the cDNA fragment of AtSSIV into the vector was carried out respecting the reading frame marked for the gene coding for GST, allowing translational fusion of the AtSSIV polypeptide fragment with the carboxy-terminal of the GST protein. The construction was confirmed by sequencing the DNA and the strain E. coli BL21 (DE3) was transformed with it.

Then, the expression and purification of the GST-SSIV fusion polypeptide was carried out with Glutathione-Agarose and the subsequent purification of the AtSSIV polypeptide fragment from GST by cleavage with thrombin and binding of the GST to a glutathione matrix. The expression of pGEX-4T3_FragSSIV took place by the addition of 1 mM isopropyl-D-thiogalactopyranoside (IPTG) in 100 ml cell culture when the optical density of the culture was 0.6. After 2 additional hours of culture, the cells were centrifuged at 10,000 g for 5 minutes, resuspended in 50 mM HEPES (pH 7.0) and sonicated. The supernatant containing the recombinant AtSSIV fragment fused with GST (GST-SSIV) was passed through a Glutathione Sepharose (GE Healthcare) affinity column. After washing the column to remove the unbound proteins, the SSIV fragment was eluted by treatment with thrombin, which cleaves the bond of the SSIV fragment with the GST protein, the latter remaining bound to the affinity column. The fragment of purified recombinant AtSSIV was mixed with Freund's complete adjuvant (in a ratio of 50/50) and then distributed into three equal aliquots. They were sent to the Animal Production and Experimentation Centre of Seville University, where rabbit polyclonal antibodies were obtained against this polypeptide. Finally, the anti-SSIV antibody was purified by FPLC using a Protein A Sepharose column (Amersham Bioscience).

### Obtaining transgenic plants that over-express AtSSIV

The constitutive over-expression of AtSSIV required the production of a binary plasmid, the production process of which is illustrated in Figure 4. AtSSIV was amplified by PCR using pAtSSIV and then cloned into pDONR/Zeo, giving rise to the pDONR/Zeo-AtSSIV plasmid. Using pDONR/Zeo-AtSSIV and pK2GW7,0 (14), the pK2GW7,0-AtSSIV (or pKan-35S-AtSSIV) plasmid was obtained, which has the 35S constitutive promoter, AtSSIV and the 35S terminator. pK2GW7,0-AtSSIV was introduced into A. tumefaciens by electroporation, giving rise to the DSM 19675 strain, which was deposited in the "German National Resource Centre for Biological Material" on 18 September 2007, address: DMSZ, Mascheroder Weg 1b D-38124 (Braunschweig, Germany). This strain was used to transform potato and Arabidopsis plants following the protocols described in the literature (15, 16).

### Obtaining AgIgAP and AgIgCAP Escherichia coli cells that over-express AtSSIV

The sequence of AtSSIV coding for the mature AtSSIV protein was amplified by PCR starting from pAtSSIV and later cloned into pET-45b(+) (Novagen) giving rise to the pET-AtSSIV plasmid as shown in Figure 5. pET-AtSSIV was introduced by electroporation into BL21(DE3) AgIgAP and AgIgCAP E. coli strains (17). These strains do not have glycogen synthase activity that could interfere with the SS activity. The over-expression of AtSSIV took place by the addition of 1 mM isopropyl-D-thiogalactopyranoside (IPTG) in 100 ml cell culture when the optical density of the culture was 0.6. After 2 additional hours of culture, the cells were centrifuged at 10,000 g for 5 minutes, resuspended in 50 mM HEPES (pH 7.0) and sonicated.

### Identification of SSIV

SSIV is a SS (EC 2.4.1.21) that transfers glucose from ADP-Glucose to the end of a starch or glycogen chain (or other type of polysaccharide consisting of glucose molecules bound to each other by α-(1,4) type covalent bonds) by the creation of a new α-(1,4) type bond. It also has the unusual feature of using maltotriose as substrate. The identification of SSIV can be achieved by any of the following ways: (a) by zymograms, (b) by analysis of the incorporation of radioactivity from radioactively labelled ADP-Glucose into glucose polysaccharides, (c) by complementation of the "glycogen-less" phenotype of the AgIgAP strain of E. coli, (d) by immunoblots making use of specific antibodies against AtSSIV and (e) by confocal microscopy analysis of the subcellular localisation of SSIV fused with the green fluorescence protein (GFP).
- By zymograms: SSIV electrophoretically separated on a native gel (50 mM GlyGly/NaOH, pH 9; 100 mM (NH4)2SO4; (5 mM 3-mercaptoetanol; 5 mM MgCl2, 0.25 g/l BSA) containing glycogen (or any other type of polysaccharide of glucose molecules bound by α-(1,4) bonds between them) and which has been incubated in a solution with ADP-Glucose will give rise to dark bands in Lugol's solution (0.5% I2/1.5% KI). The staining is due to the affinity of Lugol's iodine for long chain glucose polymers.
- By measurement of the radioactivity of glucose polymers generated from radioactively labelled ADP-Glucose: SSIV incubated as described in (3) with radioactively labelled ADP-Glucose in a solution of 50 mM glycine/NaOH (pH 9.0), 100 mM (NH4)2SO4, (5 mM 3-mercaptoethanol, 5 mM MgCl2 containing maltotriose (10 mg/ml), 1mg/ml glycogen or any other type of long polysaccharide of glucose molecules bound together with α-(1,4) bonds) will give rise to a radioactively labelled glucose polymer as a result of the incorporation of the radioactively labelled glucose from ADP-Glucose. The radioactivity incorporated in such a polymer can be measured by using a scintillation counter.
- By complementation of the "glycogen-less" phenotype of the AgIgAP strain of E. coli: the AgIgAP insertion mutant of E. coli does not accumulate glycogen as it does not have the gIgA gene coding for GIgA. This enzyme is responsible for the synthesis of glycogen from ADP-Glucose found in the cell. Therefore, the identification of SSIV activity in AgIgAP cells of E. coli is manifest by the observation of the accumulation of glycogen in the mutant transformed with pET-AtSSIV.
- By Western Blot: in the case of potato plants, AtSSIV is detected by use of the specific anti-AtSSIV antibody by the Western Blot method described in (18). In the case of Arabidopsis, the antigen-antibody complex is detected by incubation with a secondary rabbit anti-IgG conjugated with peroxidase and using the ECL Advanced® detection kit, (Amersham), which gives rise to a chemoluminescent product. The light signal is detected and quantified by a Bio-Rad ChemiDoc image capture system using "Quantity One" image analysis software also from Bio-Rad.
- By analysis of its subcellular localisation by confocal microscopy: potato and/or Arabidopsis plants were transformed with the AtSSIV-GFP chimeric construction obtained as illustrated in Figure 6. The plants were subjected to confocal microscopy observation to identify the subcellular location of the GFP fluorescence.

### Determination of the soluble sugar and starch content

Leaves and tubers were crushed in a mortar with liquid nitrogen. The starch was quantified by a spectrophotometric method consisting of the total degradation of the starch to glucose residues by the action of the amyloglucosidase enzyme and subsequent quantification of the glucose using an enzyme assay coupled with hexokinase and glucose-6-phosphate dehydrogenase enzymes (7). The amylose/amylopectin balance was determined by a spectrophotometric method (19).

### DEPOSIT OF MICROORGANISMS ACCORDING TO THE BUDAPEST TREATY

The microorganisms used in the present invention were deposited in the "German National Resource Centre for Biological Material" on 18 September 2007, at DMSZ, Mascheroder Weg 1b D-38124 (Braunschweig, Germany) with deposit number DSM 19675.

### EXAMPLES OF EMBODIMENT OF THE INVENTION

Examples are given below showing in detail the process for obtaining transgenic Arabidopsis and potato plants with a high starch content, high yield and high biomass productivity as a consequence of the increase in SSIV activity. The modes of embodiment, examples and figures that follow are provided for illustration purposes only and are not limiting of the present invention.

### Example 1: Obtaining complete cDNA coding for AtSSIV

Knowledge of the nucleotide sequence of the AtSSIV gene coding for AtSSIV enabled the creation of two specific primers, the sequences of which in the 5'-3' direction are SEQ ID NO: 1 and SEQ ID NO: 2. Making use of these primers and of RNA from Arabidopsis leaves, a complete cDNA for AtSSIV (At4g18240) was amplified by conventional RT-PCR methods and was cloned into pGEM-T easy (Promega) (Figure 1).

The nucleotide sequences of the amplified DNA and the amino acid sequence deduced are shown in SEQ ID NO: 3 and SEQ ID NO: 4 respectively.

### Example 2: Identification of the product with SSIV activity

- Zymogram identification: 100 µg of protein from crude extracts of E. coli BL21(DE3) AgIgCAP cells transformed with pET-45b(+) or with pET-AtSSIV were subjected to electrophoresis in native conditions on a 7.5% polyacrylamide gel without SDS, which contained 0.3% (p/v) of glycogen from pig liver (SIGMA). After incubating the gel overnight at ambient temperature in 50 mM GlyGly/NaOH pH 9; 100 mM (NH4)2SO4; (5 mM 3-mercaptoethanol; 5 mM MgCl2, 0.25 g/l BSA and 1 mM ADP-Glucose), it was incubated in an iodine solution (Lugol) composed of 0.5% 12/1.5% KI. The presence of new glycogen chains was revealed owing to the appearance of a dark band in the gel. This dark band is due to the affinity of Lugol for long chain glucose polymers, so that where a SS had stopped its migration and had elongated polyglucan chains by the addition of glucose residues with α-(1,4) bonds, a dyed and darker area was seen in the gel. As can be seen in the zymogram of Figure 7, BL21(DE3) AgIgCAP cells transformed with pET-AtSSIV showed a glycogen elongating activity dependent on ADP-Glucose. This activity was absent in BL21(DE3) transformed with pET-45(+).
- Identification by incorporation of radioactivity from radioactive ADP-Glucose (Figure 8). The purified enzymes were incubated at 30 °C for 30 min in 100 µl of the following reaction mixture: 50 mM Glygly/NaOH pH 9; 100 mM (NH4)2SO4; 5 mM 3-mercaptoetanol; 5 mM MgCl2; 0.25 g/l BSA; 1 mM ADP-[U- C] Glucose (3.7 GBq/mol). Lastly, 10 mg/ml of malto-oligosaccharide (with a degree of polymerisation of between 2 and 7) or corn amylopectin were added, depending on the substrate analysed. The reaction was stopped by boiling the sample for 10 min and the glucans produced were elongated by incubation at 30 °C overnight with 7.5 U of rabbit phosphorylase a (Sigma) in the presence of 50 mM Glucose-1-P (final concentration). The reaction was stopped by the addition of 3.5 ml of a solution of 75% methanol and 1% KCI and then centrifuged to precipitate the synthesised glucan. The pellet obtained was washed three times with the same stopping solution and finally the incorporated radioactivity was quantified by the addition of 5 ml of Ready Protein scintillation liquid (Beckman) followed by reading in a scintillation counter model LS 6000 IC (Beckman). The elongation with phosphorylase was omitted when the substrate used was amylopectin. As can be seen in Figure 8, assays of substrate specificity of SSIV showed that SSIV is capable of transferring glucose molecules from ADP-Glucose to polyglucans such as amylopectin. Malto-oligosaccharides of 4, 5, 6 or 7 glucose units were not good substrates for SSIV. Surprisingly, maltotriose is an excellent substrate for SSIV (as good as amylopectin). This substrate specificity pattern distinguishes SSIV from other SSs because, as can be seen in Figure 8, SSI, SSII and SSIII do not act efficiently on maltotriose (3).
- Identification by complementation of the "glycogen-less" phenotype of the AgIgAP strain of E. coli: as can be seen in Figure 9A, the AgIgAP cells of E. coli do not accumulate glycogen as they do not have GIgA. The "glycogen-less" phenotype of this strain disappears on ectopically expressing the gIgA gene coding for E. coli GIgA (Figure 9B). In the same way that AgIgAP cells of E. coli transformed with pET-glgA accumulated glycogen, so did the AgIgAP cells of E. coli transformed with pET-AtSSIV (Figure 9C, 9D).
- Identification by subcellular localisation: potato and/or Arabidopsis plants were transformed with the chimeric AtSSIV-GFP construction obtained as illustrated in Figure 6. The plants were subjected to analysis of the subcellular localisation of GFP fluorescence by D-Eclipse C1 confocal microscope (NIKON) equipped with an Ar 488 excitation laser, a BA515/30 filter for green emission, a BA650LP filter for red emission and a light detector. In the photographs of Figure 10, in contrast to what occurs with other members of the soluble starch synthase family, SSIV-GFP is bound to starch granules. Equally, the cellular localisation of SSIV in potato plant tubers transformed with the chimeric AtSSIV-GFP construction was analysed. In the photographs of Figure 16, it can be seen that SSIV-GFP is localised in the poles of the starch granules present in the amyloplasts of the potato plant tubers transformed according to the invention.

These methods of SSIV identification demonstrate that the AtSSIV protein is a SS with glucosyl transferase activity from the donor ADP-Glucose molecule to long chain polyglucan chains such as amylopectin, amylose and glycogen. Also, in contrast to the other SSs, SSIV is able to add glucose units to maltotriose. Finally, SSIV is the only member of the soluble starch synthase family that is associated with the starch granule.

### Example 3: Obtaining and characterisation of transgenic plants that over-express SSIV

Using the Agrobacterium tumefaciens DSM 19675 strain (that contains the pK2GW7,0-AtSSIV plasmid, alternatively designated as pKan-35S-AtSSIV), transgenic Arabidopsis thaliana and potato (Solanum tuberosum) plants were obtained that over-expressed AtSSIV in a constitutive way.
In order to demonstrate that the transgenic plants transformed with the A. tumefaciens DSM 19675 strain included a single insertion of the construction described above, a Southern Blot was performed on these transformed plants. The probe used for detection by this technique was created by radioactively labelling the gene conferring resistance to kanamycin with the isotope dCTP 32P. As can be seen in Figure 17, all transgenic potato plant lines (2, 7, 8 and 9) showed a single insertion of the pKan-35S-AtSSIV construction in their genome, while non-transformed control plants did not have this construction in their genome.

Compared with non-transformed plants, the plants over-expressing AtSSIV accumulated significantly higher levels of a protein of approximately 112 kDa that is recognised by the specific polyclonal antibody against AtSSIV (Figure 11). In the case of the potato, this protein has internal breaks that give rise to fragments of approximately 80 and 100 kDa. In Figure 13, it can be seen that the levels of starch in the leaves of Arabidopsis plants over-expressing AtSSIV, cultivated in greenhouse conditions, are significantly higher than those of non-transformed control plants (WT). A positive correlation can also be seen between the levels of expression of SSIV (Figure 11C) and the levels of starch in tubers of plants grown in greenhouse conditions. In addition, Arabidopsis plants cultivated in greenhouse conditions showed higher yield in the production of biomass and growth to that observed in non-transformed control plants (Figure 15 A) while their morphology was similar to that of non-transformed plants (Figure 15 B).

In addition to greenhouse cultivation, transgenic potato plants over-expressing the AtSSIV gene and control plants were cultivated in field conditions. These cultivations were performed between May and September 2009 on a plot of district 25 in Sartaguda (Navarre, Spain). The plants were distributed at random on 50 square meter plots, using 30 plants per line. The separation between rows was 90 cm. The separation between plants on the same row was 35 cm.

As can be seen in Figure 14, the potato plant tubers that over-expressed AtSSIV, cultivated in field conditions, accumulated significantly higher levels of starch than the corresponding organs of control plants. These data correlate with the significant increase in the concentration of starch found in the leaves of the transgenic plants of the invention compared to control plants cultivated in greenhouse conditions.

Table 1 shows the starch content of tubers of plants that over-expressed AtSSIV and the tubers of control plants, both of them cultivated in field conditions. The results shown in this table are the average and standard deviation of 30 different plants per line. The significantly different values to those recorded in control plants are indicated in bold. The results shown in Table 1 demonstrate that the tubers of potato plants that over-express AtSSIV showed a significant increase, approximately of 30%, in the concentration of starch expressed as a percentage of dry weight (% DW) compared to the tubers of non-transformed control plants (WT).
The productivity data per unit of area (kg/ha) shown in Table 1 indicate that the tubers of transgenic plants over-expressing AtSSIV showed a significant increase in starch content compared to the tubers of control plants (WT).

Also, the tubers of transgenic plants over-expressing AtSSIV, cultivated in field conditions, produced significantly higher concentrations of starch than the tubers of control potato plants (WT). While the tubers of control plants produced 94.65 g of starch per plant, the plants over-expressing AtSSIV produced between 103.9 and 137 g of starch per plant.

**Table 1. Quantitative parameters of transgenic plants over-expressing AtSSIV in field conditions.**

| **35S-AtSIV** | | | | |
|---|---|---|---|---|
| | **WT** | **SS4-7** | **SS4-8** | **SS4-9** |
| Tuber starch (% FW) | 11.3 ± 0,3 | **15.3 ± 0.8** | **13.2 ± 0.3** | **13.7 ± 0.5** |
| Tuber starch (% DW) | 56.1 ± 9.8 | **80.2 ± 4,5** | **72.5 ± 1.8** | **70.1 ± 2.5** |
| Tuber starch (g/plant) | 94.6 ± 1.2 | **137 ± 2.9** | **98.6 ± 1.4** | **104 ± 2.0** |
| Tuber starch (kg/ha) | 4254 ± 52 | **6091 ± 108** | 4381 ± 160 | **4619 ± 197** |

| | | | | |
|---|---|---|---|---|
| FW: fresh weight; DW: dry weight. The significantly different values to those recorded in control plants are indicated in bold. | | | | |

As can be seen in Figure 18, the amylose/amylopectin balance expressed in percentage of amylose in the tubers of transgenic plants over-expressing AtSSIV was similar to that observed in the tubers of non-transformed control plants. Therefore, although over-expression of AtSSIV brings about an increase in the quantity of starch accumulated in the tubers of potato plants that over-express AtSSIV, the quality of the starch of these transgenic tubers was similar to the quality of the starch in the tubers of non-transformed control plants. That is, the same type of starch is found both in wild non-transformed plants as in the transformed plants of the invention.

Another of the characteristics defining the transgenic plants of the invention is that they show an increase in soluble sugar content, such as glucose, fructose and sucrose (Figure 19), in the tubers compared to non-transformed control plants (WT).

Potato tubers act as reservoirs of starch and proteins. Figure 20 shows that the protein content of tubers of potato plants over-expressing AtSSIV is similar to that found in the tubers of non-transformed control potato plants. Therefore, this figure demonstrates that the over-expression of AtSSIV in transgenic plants does not alter the protein content of the tubers of these transgenic plants. This demonstrates the specificity of the over-expression of AtSSIV for the specific accumulation of starch in these transgenic plants compared to non-transformed control plants.

### REFERENCES

1. Ball, S., and Morell, M. (2003). From bacterial glycogen to starch: understanding the biogenesis of the plant sttarch granule. Annu. Rev. Plant Biol. 54, 207-233.
2. Ral, J.P., Derelle, E., Ferraz, C., Wattebled, F., Farinas, B., Corellou, F., Buléon, A., Slomianny, M.C., Delvalle, D., d'Hulst, C., Rombauts, S., Moreau, H., Ball, S. (2004). Starch division and partitioning. A mechanism for granule propagation and maintenance in the picophytoplanktonic green alga Ostreococcus tauri. Plant Physiol. 136, 3333-3340.
3. Delvallé, D., Dumez, S., Wattlebled, F., Roldán, I., Planchot, V., Berbezy, P., Colonna, P., Vyas, D., Chatterjee, M., Ball, S., Merida, A., D'Hulst, C. (2005). Soluble starch synthase I: a major determinant for the synthesis of amylopectin in Arabidopsis thaliana leaves. Plant J. 43, 398-412.
4. Hirose, T., Terao, T. (2004). A comprehensive expression analysis of the starch synthase gene family in rice (Oriza sativa L.). Planta 220, 9-16.
5. Leterrier, M., Holappa, L.D., Broglie, K.E., Beckles, D.M. (2008). Cloning, characterization and comparative analysis of a starch synthase IV gene in wheat: functional and evolutionary implications. BMC Plant Biol. Doi: 10.1186/1471-2229-8-98.
6. Zhang, X., Szydlowski, N., Delvalle, D., D'Hulst, C., James, M.G., Myers, A.M. (2008). Overlapping functions of the starch synthases SSII and SSIII in amylopectin biosynthesis in Arabidopsis. BMC Plant Biol. doi:10.1186/1471-2229-8-96.
7. Roldán, I., Wattebled, F., Lucas, M.M., Delvallé, D., Planchot, V., Jiménez, S., Pérez, R., Ball, S., D'Hulst, C., and Mérida, A. (2007). The phenotype of soluble starch synthase IV defective mutants of Arabidopsis thaliana suggests a novel function of elongation enzymes in the control of starch granule formation. The Plant Journal 49:492-504.
8. Edwards, A., Fulton, D.C., Hylton, C.M., Jobling, S.A., Gidley, M., Rössner, U., Martín, C., Smith, A.M. (1999). A combined reduction in activity of starch synthases II and III of potato has novel effects on the starch of tubers. Plant J. 17, 251-261.
9. Jobling, S.A., Westcott, R.J., Tayal, A., Jeffcoat, R., Schwall, G.P. (2002). Production of a freeze-thaw-stable potato starch by antisense inhibition of three starch synthase genes. Nat. Biotechnol 20, 295-299.
10. Shewmaker, C.K., Boyer, C.D., Wiesenborn, D.P., Thompson, D.B., Boersig, M.R., Oakes, J.V., Stalker, D.M. (1994). Expression of Escherichia coli glycogen synthase in the tubers of transgenic potatoes results in a highly branched starch. Plant Physiol. 104, 1159-1166.
11. Lloyd, J.R., Landschütze, V., Kossmann, J. (1999). Simultaneous antisense inhibition of two starch-synthase isoforms in potato tubers leads to accumulation of grossly modified amylopectin. Biochem. J. 338, 515-521.
12. Abel, G.J.W., Springer, F., Willmitzer, L., Kossmann, J. (1996). Cloning and functional analysis of a cDNA encoding a novel 139 kDa starch synthase from potato (Solanum tuberosum L.). Plant J. 10, 981-991.
13. Zhang, X., Myers, A.M., James, M.G. (2005). Mutations affecting starch synthase III in Arabidopsis alter leaf starch structure and increase the rate of starch synthesis. Plant Physiol. 138, 663-674.
14. Karimi, M., Inze, D., Depicker, A. (2002). GATEWAY™ vectors for Agrobacterium-mediated plant transformation. Trends Plant Sci. 7: 193-195.
15. Rocha-Sosa, M., Sonnewald, U., Frommer, W., Stratmann, M., Schell, J., Willmitzer, L. (1989). Both developmental and metabolic signals activate the promoter of a class I patatine gene. EMBO J. 8, 23-29.
16. Clough, S.J., Bent, A.F. (1998). Floral dip: a simplified method for Agrobacterium-mediated transformation of Arabidopsis thaliana. Plant J. 16: 735-43.
17. Moran-Zorzano, M.T., Alonso-Casajus, N., Muñoz, F.J., Viale, A.M., Baroja-Fernández, Eydallin G., Pozueta-Romero, J. (2007). Occurrence of more than one important source of ADPglucose linked to glycogen biosynthesis in Escherichia coli and Salmonella enterica. FEBS Lett. 581, 4423-4429.
18. Towbin, H., Staehelin, T., Gordon, J. (1979). Electrophoretic transfer of proteins from polyacrylamide gels to nitrocellulose sheets: procedures and some applications. Proc. Natl. Acad. Sci. USA 76, 4350-4354.
19. Andersson, M., Melander, M., Pojmark, P., Larsson, H., Bülow, L., Hofvander, P. (2006). Targeted gene suppression by RNA interference: an efficient method for production of high-amylose potato lines" J. Biotechnol. 123, 137-148.

## Claims

1. Process for obtaining transgenic plants with a content of starch at least 10% higher than the content found in non-transformed plants (WT), **characterised by** the transformation of non-transformed plants (WT) with an expression vector comprising a nucleotide sequence encoding and over-expressing for an enzyme with SSIV activity represented by SEQ ID NO:4.

2. Process according to claim 1, wherein the transgenic plants obtained contain at least 10% biomass higher than non-transformed plants (WT).

3. Process according to claim 1 or 2, **characterised in that** the level of SSIV expression in transgenic plants is at least twice the level of expression of SSIV in the non-transformed plant (WT).

4. Process according to any of the claims 1 to 3 wherein the enzyme with SSIV activity represented by SEQ ID NO:4 is encoded by a nucleotide sequence comprised in the expression vector used for transforming the non-transformed plant (WT), which comprises SEQ ID NO:3.

5. Plant cell transformed with an expression vector that comprises a nucleotide sequence encoding and over-expressing for a protein with SSIV activity represented by SEQ ID NO:4 and having a content of starch at least 10% higher than the content found in non-transformed plant (WT) cells.

6. Plant cell according to claim 5 containing at least 10% biomass higher than non-transformed plants (WT).

7. Plant cell according to claim 5 or 6, **characterised in that** the nucleotide sequence, comprised in the expression vector used for transforming the non-transformed plant (WT) cells, comprises SEQ ID NO:3.

8. Plant cell according to any of the claims 5 to 7 **characterised in that** it is a plant cell belonging to a plant species selected from: potato (*Solanum tuberosum),* tobacco *(Nicotiana tabacum),* barley *(Hordeum vulgare),* rice (*Oryza sativa),* corn *(Zea mays*) or Arabidopsis *(Arabidopsis thaliana*).

9. Transgenic plant transformed with an expression vector that comprises a nucleotide sequence encoding and over-expressing for a protein with SSIV activity represented by SEQ ID NO:4, and having a content of starch at least 10% higher than the content found in non-transformed plants (WT).

10. Transgenic plant according to claim 9 containing at least 10% biomass higher than non-transformed plants (WT).

11. Transgenic plant according to claims 9 or 10 **characterised in that** it shows a level of expression of SSIV of at least twice that observed in the non-transformed plants (WT).

12. Transgenic plant according to any of the claims 9 to 11 **characterised in that** the nucleotide sequence, comprised in the expression vector used for transforming the non-transformed plant (WT), comprises SEQ ID NO:3.

13. Transgenic plant according to claims 9 to 12, selected from the group comprising: potato *(Solanum tuberosum),* tobacco *(Nicotiana tabacum*), barley *(Hordeum vulgare*), rice (*Oryza sativa),* corn *(Zea mays*) or Arabidopsis *(Arabidopsis thaliana*).

14. Use of the transgenic plants of claims 9 to 13; or of the transformed plant cells of claims 5 to 8, for the production of carbohydrates selected from a group including: starch, glucose, fructose and sucrose.

15. Use of the transgenic plants of claims 9 to 13; or of the transformed plant cells of claims 5 to 8, for the production of biomass.

## Patentansprüche

1. Verfahren zur Herstellung transgener Pflanzen mit einem Stärkegehalt, der mindestens 10 % höher ist als der in nicht transformierten Pflanzen (WT) vorliegende Gehalt, **gekennzeichnet durch** die Transformation nicht transformierter Pflanzen (WT) mit einem Expressionsvektor, der eine Nukleotidsequenz umfasst, die für ein **durch** die SEQ ID NO: 4 dargestelltes Enzym mit SSIV-Aktivität kodiert und es überexprimiert.

2. Verfahren nach Anspruch 1, wobei die erhaltenen transgenen Pflanzen mindestens 10 % mehr Biomasse enthalten als die nicht transformierten Pflanzen (WT).

3. Verfahren nach Anspruch 1 oder 2, **dadurch gekennzeichnet, dass** das SSIV-Expressionsniveau in transgenen Pflanzen mindestens doppelt so hoch ist wie das SSIV-Expressionsniveau in der nicht transformierten Pflanze (WT).

4. Verfahren nach einem der Ansprüche 1 bis 3, wobei das durch die SEQ ID NO: 4 dargestellte Enzym mit SSIV-Aktivität von einer Nukleotidsequenz kodiert wird, die im zur Transformation der nicht transformierten Pflanze (WT) eingesetzten Expressionsvektor enthalten ist und die SEQ ID NO: 3 umfasst.

5. Pflanzenzelle, die mit einem Expressionsvektor transformiert ist, der eine Nukleotidsequenz umfasst, die für ein durch die SEQ ID NO: 4 dargestelltes Protein mit SSIV-Aktivität kodiert und es überexprimiert, und die einen Stärkegehalt aufweist, der mindestens 10 % höher ist als der in nicht transformierten Pflanzenzellen (WT) vorliegende Gehalt.

6. Pflanzenzelle nach Anspruch 5, die mindestens 10 % mehr Biomasse enthält als die nicht transformierten Pflanzen (WT).

7. Pflanzenzelle nach Anspruch 5 oder 6, **dadurch gekennzeichnet, dass** die Nukleotidsequenz, die im zur Transformation der nicht transformierten Pflanzenzellen (WT) eingesetzten Expressionsvektor enthalten ist, die SEQ ID NO: 3 umfasst.

8. Pflanzenzelle nach einem der Ansprüche 5 bis 7, **dadurch gekennzeichnet, dass** sie eine Pflanzenzelle ist, die einer Pflanzenart angehört, die ausgewählt wird aus: Kartoffel (*Solanum tuberosum*), Tabak *(Nicotiana tabacum),* Gerste *(Hordeum vulgare),* Reis (*Oryza sativa),* Mais (*Zea mays*) oder Schaumkresse *(Arabidopsis thaliana*).

9. Transgene Pflanze, die mit einem Expressionsvektor transformiert ist, der eine Nukleotidsequenz umfasst, die für ein durch die SEQ ID NO: 4 dargestelltes Protein mit SSIV-Aktivität kodiert und es überexprimiert, und die einen Stärkegehalt aufweist, der mindestens 10 % höher ist als der in nicht transformierten Pflanzenzellen (WT) vorliegende Gehalt.

10. Transgene Pflanze nach Anspruch 9, die mindestens 10 % mehr Biomasse enthält als die nicht transformierten Pflanzen (WT).

11. Transgene Pflanze nach Anspruch 9 oder 10, **dadurch gekennzeichnet, dass** sie ein SSIV-Expressionsniveau aufweist, das mindestens doppelt so hoch ist wie das der nicht transformierten Pflanzen (WT).

12. Transgene Pflanze nach einem der Ansprüche 9 bis 11, **dadurch gekennzeichnet, dass** die Nukleotidsequenz, die im zur Transformation der nicht transformierten Pflanze (WT) eingesetzten Expressionsvektor enthalten ist, die SEQ ID NO: 3 umfasst.

13. Transgene Pflanze nach den Ansprüchen 9 bis 12, die aus der Gruppe bestehend aus Kartoffel (*Solanum tuberosum*), Tabak *(Nicotiana tabacum*), Gerste *(Hordeum vulgare),* Reis (*Oryza sativa),* Mais (*Zea mays*) oder Schaumkresse *(Arabidopsis thaliana)* ausgewählt wird.

14. Verwendung der transgenen Pflanzen der Ansprüche 9 bis 13 oder der transformierten Pflanzenzellen der Ansprüche 5 bis 8 zur Herstellung von Kohlenhydraten, die aus der Gruppe bestehend aus Stärke, Glucose, Fructose und Saccharose ausgewählt werden.

15. Verwendung der transgenen Pflanzen der Ansprüche 9 bis 13 oder der transformierten Pflanzenzellen der Ansprüche 5 bis 8 zur Herstellung von Biomasse.

## Revendications

1. Procédé d'obtention de plantes transgéniques avec un contenu d'amidon au moins 10% supérieur au contenu présent dans des plantes non transformées (WT), **caractérisé par** la transformation de plantes non transformées (WT) avec un vecteur d'expression comprenant une séquence nucléotide codant et surexprimant une enzyme avec une activité du SSIV représentée par la SEQ ID N° : 4.

2. Procédé selon la revendication 1, où les plantes transgéniques obtenues contiennent au moins un contenu de biomasse qui est 10% supérieur à celui des plantes non transformées (WT).

3. Procédé selon la revendication 1 ou 2, **caractérisé en ce que** le niveau d'expression du SSIV dans les plantes transgéniques équivaut à au moins deux fois le niveau d'expression du SSIV dans les plantes non transformées (WT).

4. Procédé selon l'une quelconque des revendications 1 à 3, où l'enzyme ayant une activité du SSIV représentée par la SEQ ID N° : 4 est codée par une séquence nucléotide comprise dans le vecteur d'expression utilisé pour la transformation des plantes non transformées (WT), qui comprend la SEQ ID N° : 3.

5. Cellule de plante transformée avec un vecteur d'expression qui comprend une séquence nucléotide codant et surexprimant une protéine avec une activité du SSIV représentée par la SEQ ID N° : 4, et qui possède un contenu d'amidon au moins 10% supérieur au contenu présent dans les cellules de plantes non transformées (WT).

6. Cellule de plante selon la revendication 5 contenant au moins un contenu de biomasse 10% supérieur à celui des plantes non transformées (WT).

7. Cellule de plante selon la revendication 5 ou 6, **caractérisée en ce que** la séquence nucléotide, comprise dans le vecteur d'expression utilisé pour la transformation des cellules de plantes non transformées (WT), comprend la SEQ ID N° : 3.

8. Cellule de plante selon l'une quelconque des revendications 5 à 7, **caractérisée en ce qu'**il s'agit d'une cellule de plante appartenant à une espèce végétale sélectionnée parmi : la pomme de terre (*Solanum tuberosum),* le tabac *(Nicotiana tabacum),* l'orge *(Hordeum vulgare),* le riz (*Oryza sativa),* le maïs *(Zea mays*) ou l'Arabidopsis *(Arabidopsis thaliana*).

9. Plante transgénique transformée avec un vecteur d'expression qui comprend une séquence nucléotide codant et surexprimant une protéine avec une activité du SSIV représentée par la SEQ ID N° : 4, et ayant un contenu d'amidon au moins 10% supérieur au contenu présent dans les plantes non transformées (WT).

10. Plante transgénique selon la revendication 9 contenant au moins un contenu de biomasse 10% supérieur à celui des plantes non transformées (WT).

11. Plante transgénique selon les revendications 9 à 10, **caractérisée en ce qu'**elle montre un niveau d'expression du SSIV équivalent à au moins deux fois le niveau observé dans la plante non transformée (WT).

12. Plante transgénique selon l'une quelconque des revendications 9 à 11, **caractérisée en ce que** la séquence nucléotide, comprise dans le vecteur d'expression utilisé pour la transformation des cellules de plantes non transformées (WT), comprend la SEQ ID N° : 3.

13. Plante transgénique selon les revendications 9 à 12, sélectionnée dans le groupe comprenant : la pomme de terre (*Solanum tuberosum),* le tabac *(Nicotiana tabacum),* l'orge *(Hordeum vulgare),* le riz (*Oryza sativa),* le maïs (*Zea mays*) ou l'Arabidopsis (*Arabidopsis thaliana*).

14. Utilisation des plantes transgéniques selon les revendications 9 à 13 ; ou des cellules de plantes transformées selon les revendications 5 à 8, pour la production d'hydrates de carbone sélectionnés parmi un groupe comprenant : de l'amidon, du glucose, du fructose et du saccharose.

15. Utilisation des plantes transgéniques selon les revendications 9 à 13 ; ou des cellules de plantes transformées selon les revendications 5 à 8, pour la production de biomasse.


### SEQUENCE LISTING

<110> IDEN BIOTECHNOLOGY, S.L.
<120> PROCESS FOR THE PRODUCTION OF TRANSGENIC PLANTS THAT HAVE A HIGH CONTENT AND YIELD OF STARCH AND BIOMASS
<130> EP-04795
<160> 6
<170> PatentIn version 3.3
<210> 1
   <211> 69
   <212> DNA
   <213> Artificial sequence
<220>
   <223> ATSSIV_Direct primer 5'- 3'
<400> 1
   ggggacaagt ttgtacaaaa aagcaggctt cgaaggagat agaaccatgg cgacgaagct 60
   atcgagctt 69
<210> 2
   <211> 54
   <212> DNA
   <213> Artificial sequence
<220>
   <223> ATSSIV_ Reverse primer 5'- 3'
<400> 2
   ggggaccact ttgtacaaga aagctgggtt cacgtgcgat taggaacagc tctt 54
<210> 3
   <211> 3123
   <212> DNA
   <213> Artificial sequence
<220>
   <223> AtASS4 5'- 3'
<400> 3
   atgacgacga agctatcgag cttctgtttc ttaacccatg gattagcagg aatctcctgc     60
   gagagagaac atggaagttc tcgtcgattt ttctatctcc cttctcgtcg attagtttct    120
   acttcgtgta agatgcgaca acaacgtggt ttcgactcta gtaaaagaca agaggtcaag    180
   aaaggctccc ctaaaccgat cctatctata aattcaggtc ttcagagcaa caatgatgag    240
   gaatctgatc tggagaatgg ttctgcggac agtgtcccga gtcttaagtc agatgctgaa    300
   aagggtagta gtattcatgg cagcatagat atgaatcatg ctgatgagaa tcttgaaaag    360
   aaagatgata tacaaacaac tgaagttact cggcgcaaga gtaaaactgc aaagaagaaa    420
   ggggagagta ttcatgctac gattgatatt ggacatgatg atgggaagaa tttagataat    480
   attactgtgc ctgaggttgc aaaagctttg tcccttaaca aaagtgaagg cgagcagatt    540
   tcagatggac agtttgggga actaatgaca atgataagaa gtgcagaaaa aaatattctt     600
   cggcttgatg aagcacgggc cactgctctt gacgacctta acaagattct tagtgataag     660
   gaagcgttgc agggagaaat caatgtcttg gaaatgaaat tgtctgagac tgatgaaagg     720
   attaaaactg ctgctcaaga gaaagcacat gtagagcttc tggaagaaca gttagagaag     780
   cttcgtcatg aaatgatctc ccctatagaa agtgatggtt atgttctagc tcttagcaaa     840
   gagcttgaaa cattgaagct ggagaatcta tctttgagaa acgatataga aatgcttaag     900
   tcagaacttg acagtgttaa agataccggt gaacgtgtgg ttgtgttgga aaaggagtgc     960
   tccggtttgg aatcttctgt aaaggatttg gaatctaagt tatcggtttc tcaggaagat    1020
   gtctcgcagc tttctactct taaaatcgaa tgcactgatc tatgggcgaa ggtagagact    1080
   ctacagctgt tgttagatag agctaccaaa caagcagagc aagcagttat agtgttacaa    1140
   cagaaccaag atctaagaaa taaggtcgac aaaattgagg aatcacttaa agaagccaac    1200
   gtttataaag aatcttcaga gaaaattcaa cagtacaatg agctaatgca acataaggtg    1260
   acattacttg aggagcggct cgaaaagtct gatgcagaga tattctcata tgttcagtta    1320
   tatcaagaat cgataaaaga attccaggaa acacttgaaa gtttgaagga agaaagcaag    1380
   aaaaaatcaa gagatgaacc ggttgatgat atgccttggg attattggag tcggttactt    1440
   ttaactgttg atggatggct gcttgaaaag aaaatagcaa gcaacgatgc tgacttactg    1500
   agagacatgg tatggaagaa agatcgaaga attcatgata cctatattga cgtcaaagat    1560
   aagaatgaac gtgatgccat ctctgcattt ctcaagcttg tctcatctcc aacaagttca    1620
   ggattgtatg tcgttcacat tgcagctgag atggcacctg tagctaaggt gggaggtttg    1680
   ggagatgttg tggcaggtct tggtaaggca ttgcaaagaa aaggtcatct ggtggagatt    1740
   attcttccca aatatgactg tatgcagtat gatcgcgtgc gtgacttaag ggctttggat    1800
   actgttgtgg agtcatattt tgatgggaag ttatataaaa acaaaatctg gattggcact    1860
   gttgaaggtt tacctgtaca tttcattgaa cctcaacatc caagcaaatt cttctggaga    1920
   ggacagtttt atggagagca agatgatttc agacgcttct cgtattttag ccgagctgca    1980
   ctagagttgc ttcttcagtc cggcaaaaaa cccgacatca tacattgtca tgactggcaa    2040
   acagcttttg ttgcgccgct gtattgggat ctgtatgctc caaagggatt agattctgca    2100
   agaatatgct ttacatgtca taattttgag tatcaaggta ccgcgtctgc ttcagaattg    2160
   ggatcttgcg ggcttgatgt taaccagtta aatagaccag acagaatgca ggatcactcg    2220
   tctggagata gagtcaatcc tgttaagggc gctataattt tctcaaacat tgtaacaacc    2280
   gtgtccccta cttatgcaca agaagttcga acagctgagg gaggaaaagg actccattca    2340
   acactcaatt ttcactccaa gaaattcatt ggaatcctca acggcattga cacagattca    2400
   tggaatcctg ccaccgaccc cttcctcaag gctcagttca acgctaaaga tctacaaggg    2460
   aaagaagaaa acaaacacgc ccttagaaag cagcttggac tttcttcagc ggagtcaaga   2520
   cggcctttgg ttggttgcat aacaagatta gtaccacaga aaggagttca tctaatcaga   2580
   catgccatat acagaacatt agagttaggt ggacaatttg tacttcttgg ttctagcccg   2640
   gttccacata ttcagaggga atttgaaggt attgaacaac agtttaaaag ccatgatcat   2700
   gtccggttgt tactgaagta cgatgaagct ctgtctcata cgatttacgc agcctccgat   2760
   ttgttcatca ttccgtcaat ttttgagcct tgtggactta cacagatgat cgctatgaga   2820
   tatggctcca ttccaattgc gcgaaaaact ggaggcttaa atgacagtgt cttcgacatt   2880
   gatgatgata caataccaac acagtttcaa aatggattta catttcaaac cgcggacgaa   2940
   cagggtttca attatgcgtt ggagcgagca tttaatcact acaagaaaga tgaagagaaa   3000
   tggatgagac ttgtagagaa agtgatgagt atagatttca gctggggatc atcagctaca   3060
   caatacgaag aactctacac gagatcagtg tccagagcaa gagctgttcc taatcgcacg   3120
   tga                                                                 3123
<210> 4
   <211> 1040
   <212> PRT
   <213> Artificial sequence
<220>
   <223> AtASS4
<400> 4
   Met Thr Thr Lys Leu Ser Ser Phe Cys Phe Leu Thr His Gly Leu Ala
   1               5                   10                  15
   Gly Ile Ser Cys Glu Arg Glu His Gly Ser Ser Arg Arg Phe Phe Tyr
               20                  25                  30
   Leu Pro Ser Arg Arg Leu Val Ser Thr Ser Cys Lys Met Arg Gln Gln
           35                  40                  45
   Arg Gly Phe Asp Ser Ser Lys Arg Gln Glu Val Lys Lys Gly Ser Pro
       50                  55                  60
   Lys Pro Ile Leu Ser Ile Asn Ser Gly Leu Gln Ser Asn Asn Asp Glu
   65                  70                  75                  80
   Glu Ser Asp Leu Glu Asn Gly Ser Ala Asp Ser Val Pro Ser Leu Lys
                   85                  90                  95
   Ser Asp Ala Glu Lys Gly Ser Ser Ile His Gly Ser Ile Asp Met Asn
               100                 105                 110
   His Ala Asp Glu Asn Leu Glu Lys Lys Asp Asp Ile Gln Thr Thr Glu
           115                 120                 125
   Val Thr Arg Arg Lys Ser Lys Thr Ala Lys Lys Lys Gly Glu Ser Ile
   130 135 140
   His Ala Thr Ile Asp Ile Gly His Asp Asp Gly Lys Asn Leu Asp Asn
   145 150 155 160
   Ile Thr Val Pro Glu Val Ala Lys Ala Leu Ser Leu Asn Lys Ser Glu
   165 170 175
   Gly Glu Gln Ile Ser Asp Gly Gln Phe Gly Glu Leu Met Thr Met Ile
   180 185 190
   Arg Ser Ala Glu Lys Asn Ile Leu Arg Leu Asp Glu Ala Arg Ala Thr
   195 200 205
   Ala Leu Asp Asp Leu Asn Lys Ile Leu Ser Asp Lys Glu Ala Leu Gln
   210 215 220
   Gly Glu Ile Asn Val Leu Glu Met Lys Leu Ser Glu Thr Asp Glu Arg
   225 230 235 240
   Ile Lys Thr Ala Ala Gln Glu Lys Ala His Val Glu Leu Leu Glu Glu
   245 250 255
   Gln Leu Glu Lys Leu Arg His Glu Met Ile Ser Pro Ile Glu Ser Asp
   260 265 270
   Gly Tyr Val Leu Ala Leu Ser Lys Glu Leu Glu Thr Leu Lys Leu Glu
   275 280 285
   Asn Leu Ser Leu Arg Asn Asp Ile Glu Met Leu Lys Ser Glu Leu Asp
   290 295 300
   Ser Val Lys Asp Thr Gly Glu Arg Val Val Val Leu Glu Lys Glu Cys
   305 310 315 320
   Ser Gly Leu Glu Ser Ser Val Lys Asp Leu Glu Ser Lys Leu Ser Val
   325 330 335
   Ser Gln Glu Asp Val Ser Gln Leu Ser Thr Leu Lys Ile Glu Cys Thr
   340 345 350
   Asp Leu Trp Ala Lys Val Glu Thr Leu Gln Leu Leu Leu Asp Arg Ala
   355 360 365
   Thr Lys Gln Ala Glu Gln Ala Val Ile Val Leu Gln Gln Asn Gln Asp
   370 375 380
   Leu Arg Asn Lys Val Asp Lys Ile Glu Glu Ser Leu Lys Glu Ala Asn
   385                 390                 395                 400
   Val Tyr Lys Glu Ser Ser Glu Lys Ile Gln Gln Tyr Asn Glu Leu Met
                   405                 410                 415
   Gln His Lys Val Thr Leu Leu Glu Glu Arg Leu Glu Lys Ser Asp Ala
               420                 425                 430
   Glu Ile Phe Ser Tyr Val Gln Leu Tyr Gln Glu Ser Ile Lys Glu Phe
           435                 440                 445
   Gln Glu Thr Leu Glu Ser Leu Lys Glu Glu Ser Lys Lys Lys Ser Arg
       450                 455                 460
   Asp Glu Pro Val Asp Asp Met Pro Trp Asp Tyr Trp Ser Arg Leu Leu
   465                 470                 475                 480
   Leu Thr Val Asp Gly Trp Leu Leu Glu Lys Lys Ile Ala Ser Asn Asp
                   485                 490                 495
   Ala Asp Leu Leu Arg Asp Met Val Trp Lys Lys Asp Arg Arg Ile His
               500                 505                 510
   Asp Thr Tyr Ile Asp Val Lys Asp Lys Asn Glu Arg Asp Ala Ile Ser
           515                 520                 525
   Ala Phe Leu Lys Leu Val Ser Ser Pro Thr Ser Ser Gly Leu Tyr Val
       530                 535                 540
   Val His Ile Ala Ala Glu Met Ala Pro Val Ala Lys Val Gly Gly Leu
   545                 550                 555                 560
   Gly Asp Val Val Ala Gly Leu Gly Lys Ala Leu Gln Arg Lys Gly His
                   565                 570                 575
   Leu Val Glu Ile Ile Leu Pro Lys Tyr Asp Cys Met Gln Tyr Asp Arg
               580                 585                 590
   Val Arg Asp Leu Arg Ala Leu Asp Thr Val Val Glu Ser Tyr Phe Asp
           595                 600                 605
   Gly Lys Leu Tyr Lys Asn Lys Ile Trp Ile Gly Thr Val Glu Gly Leu
       610                 615                 620
   Pro Val His Phe Ile Glu Pro Gln His Pro Ser Lys Phe Phe Trp Arg
   625                 630                 635                 640
   Gly Gln Phe Tyr Gly Glu Gln Asp Asp Phe Arg Arg Phe Ser Tyr Phe
                   645                 650                 655
   Ser Arg Ala Ala Leu Glu Leu Leu Leu Gln Ser Gly Lys Lys Pro Asp
               660                 665                 670
   Ile Ile His Cys His Asp Trp Gln Thr Ala Phe Val Ala Pro Leu Tyr
           675                 680                 685
   Trp Asp Leu Tyr Ala Pro Lys Gly Leu Asp Ser Ala Arg Ile Cys Phe
       690                 695                 700
   Thr Cys His Asn Phe Glu Tyr Gln Gly Thr Ala Ser Ala Ser Glu Leu
   705                 710                 715                 720
   Gly Ser Cys Gly Leu Asp Val Asn Gln Leu Asn Arg Pro Asp Arg Met
                   725                 730                 735
   Gln Asp His Ser Ser Gly Asp Arg Val Asn Pro Val Lys Gly Ala Ile
               740                 745                 750
   Ile Phe Ser Asn Ile Val Thr Thr Val Ser Pro Thr Tyr Ala Gln Glu
           755                 760                 765
   Val Arg Thr Ala Glu Gly Gly Lys Gly Leu His Ser Thr Leu Asn Phe
       770                 775                 780
   His Ser Lys Lys Phe Ile Gly Ile Leu Asn Gly Ile Asp Thr Asp Ser
   785                 790                 795                 800
   Trp Asn Pro Ala Thr Asp Pro Phe Leu Lys Ala Gln Phe Asn Ala Lys
                   805                 810                 815
   Asp Leu Gln Gly Lys Glu Glu Asn Lys His Ala Leu Arg Lys Gln Leu
               820                 825                 830
   Gly Leu Ser Ser Ala Glu Ser Arg Arg Pro Leu Val Gly Cys Ile Thr
           835                 840                 845
   Arg Leu Val Pro Gln Lys Gly Val His Leu Ile Arg His Ala Ile Tyr
       850                 855                 860
   Arg Thr Leu Glu Leu Gly Gly Gln Phe Val Leu Leu Gly Ser Ser Pro
   865                 870                 875                 880
   Val Pro His Ile Gln Arg Glu Phe Glu Gly Ile Glu Gln Gln Phe Lys
                   885                 890                 895
   Ser His Asp His Val Arg Leu Leu Leu Lys Tyr Asp Glu Ala Leu Ser
               900                 905                 910
   His Thr Ile Tyr Ala Ala Ser Asp Leu Phe Ile Ile Pro Ser Ile Phe
           915                 920                 925
   Glu Pro Cys Gly Leu Thr Gln Met Ile Ala Met Arg Tyr Gly Ser Ile
       930                 935                 940
   Pro Ile Ala Arg Lys Thr Gly Gly Leu Asn Asp Ser Val Phe Asp Ile
   945                 950                 955                 960
   Asp Asp Asp Thr Ile Pro Thr Gln Phe Gln Asn Gly Phe Thr Phe Gln
                   965                 970                 975
   Thr Ala Asp Glu Gln Gly Phe Asn Tyr Ala Leu Glu Arg Ala Phe Asn
               980                 985                 990
   His Tyr Lys Lys Asp Glu Glu Lys  Trp Met Arg Leu Val  Glu Lys Val
           995                 1000                 1005
   Met Ser  Ile Asp Phe Ser Trp  Gly Ser Ser Ala Thr  Gln Tyr Glu
       1010                 1015                 1020
   Glu Leu  Tyr Thr Arg Ser Val  Ser Arg Ala Arg Ala  Val Pro Asn
       1025                 1030                 1035
   Arg Thr
       1040
<210> 5
   <211> 25
   <212> DNA
   <213> Artificial sequence
<220>
   <223> Oligonucleotide 5'- 3'
<400> 5
   catatggaga actgatgaaa ggatt 25
<210> 6
   <211> 18
   <212> DNA
   <213> Artificial sequence
<220>
   <223> Oligonucleotide 5'- 3'
<400> 6
   ctcgagtctt tataaacg 18